Europäisches Patentamt

European Patent Office

Office européen des brevets

(11) Numéro de publication : **0 410 895 B1**

(12) # FASCICULE DE BREVET EUROPEEN

(45) Date de publication du fascicule du brevet :
**18.05.94 Bulletin 94/20**

(51) Int. Cl.[5] : **C12N 15/90,** C12N 15/56,
C12R 1/125, // C12N15/62

(21) Numéro de dépôt : **90402172.2**

(22) Date de dépôt : **27.07.90**

(54) **Procédé de production de protéine à partir de souches de bacillus subtilis et procédé de préparation desdites souches.**

(30) Priorité : **27.07.89 FR 8910143**

(43) Date de publication de la demande :
**30.01.91 Bulletin 91/05**

(45) Mention de la délivrance du brevet :
**18.05.94 Bulletin 94/20**

(84) Etats contractants désignés :
**AT BE CH DE DK ES FR GB GR IT LI LU NL SE**

(56) Documents cités :
**EP-A- 0 166 628
EP-A- 0 205 371
J. MOL. BIOL., vol. 196, 1987, ACADEMIC PRESS Inc. (London) Ltd., pages 39 - 48; NOIROT, Ph. et al.: "Plasmid replication stimulates DNA recombination in Bacillus subtilis"
APPLIED AND ENVIRONMENTAL MICROBIOLOGY, vol. 55, no. 11, novembre 1989, AMERICAN SOCIETY FOR MICROBIOLOGY, pages 2739 - 2744; JOLIFF, G. et al.: "Inducible secretion of a cellulase from Clostridium thermocellum in Bacillus subtilis."
AGRIC. BIOL. CHEM., vol. 50, no. 7, 1986, pages 1771 - 1775; FURUSATO, T. et al.: "RecE-dependent gene amplification induced by a tunicamycin-resistantmutation (tmrA7) in Bacillus subtilis."**

(73) Titulaire : **INSTITUT NATIONAL DE LA RECHERCHE AGRONOMIQUE
145, rue de l'Université
F-75341 Paris Cédex 07 (FR)**
Titulaire : **INSTITUT PASTEUR
25, rue du Docteur Roux
F-75015 Paris (FR)**

(72) Inventeur : **Joliff, Gwennael
48 rue Truffaut
F-75017 Paris (FR)**
Inventeur : **Petit, Marie-Agnès
69 rue Claude Bernard
F-75005 Paris (FR)**
Inventeur : **Mesas, Juan Manuel
E.U.I.T.A., Avenida de Madrid, 81
E-27002 Lugo (ES)**
Inventeur : **Klier, André
6 Allée Guynemer
F-93330 Neuilly sur Marne (FR)**
Inventeur : **Rapoport, Georges
20 rue de l'Armorique
F-75015 Paris (FR)**
Inventeur : **Ehrlich, Dusko Stanislas
99 boulevard Blanqui
F-75013 Paris (FR)**
Inventeur : **Looten, Phillipe
66 Allée de l'Artois
F-59130 Lambersart (FR)**
Inventeur : **Vernade, Didier
1 rue de l'Assemblée Nationale
F-78000 Versailles (FR)**

(74) Mandataire : **Warcoin, Jacques et al
Cabinet Régimbeau 26, avenue Kléber
F-75116 Paris (FR)**

EP 0 410 895 B1

## Description

La présente invention concerne un procédé de production d'une protéine déterminée chez les bactéries du genre Bacillus, en particulier Bacillus subtilis, ainsi que des souches de Bacillus, notamment Bacillus subtilis utiles dans le procédé et un procédé de préparation desdites souches.

On a décrit dans FR 84 06701 (2 563 533) un procédé de préparation d'une souche de Bacillus dans le chromosome de laquelle un gène déterminé a été amplifié, c'est-à-dire est présent à plusieurs exemplaires, dans lequel :

a) on effectue l'intégration dans le chromosome de ladite souche d'au moins une séquence d'ADN dite d'intégration comportant au moins

. une séquence d'ADN dite unité amplifiable de structure : -D-M-D-, ledit gène M et à chaque extrémité comportant deux séquences identiques D dans le sens direct, ces séquences D assurant l'intégration du gène M dans un gène non essentiel de Bacillus,

. l'unité amplifiable comportant en outre un gène sélectionnable.

b) on sélectionne ensuite les souches de Bacillus obtenues par culture sur un milieu de sélection correspondant au gène sélectionnable et on sélectionne les souches dont le génome correspond à la présence d'un nombre de copies dudit gène accrû par rapport à la population bactérienne avant sélection.

Ainsi, on a pu obtenir une unité d'amplification comportant outre la séquence dupliquée, le gène de résistance à la kanamycine (Km$^r$), le gène de résistance au chloramphénicol (Cm$^r$). Une amplification des deux gènes Km$^r$ ou Cm$^r$ a été obtenue en sélectionnant les souches résistantes à des concentrations élevées d'antibiotique. Mais aucune production de protéine n'a été décrite avec des souches selon ce procédé.

Le but de la présente invention est d'induire de plus hauts niveaux d'amplification d'un fragment du génome de Bacillus subtilis de façon stable et controlable d'une part, et d'autre part que la machinerie cellulaire soit encore capable d'assurer la transcription, la traduction de toutes les séquences ainsi amplifiées et, le cas échéant, la sécrétion des protéines codées par un gène amplifié.

Selon la présente invention, on a découvert un nouveau procédé d'amplification d'une souche de Bacillus susceptible de produire une protéine déterminée en quantité importante dans le chromosome de laquelle un gène déterminé codant ladite protéine a été amplifié. Ce procédé est caractérisé en ce que :

a) on effectue l'intégration dans le chromosome de ladite souche d'au moins une séquence d'ADN dite d'intégration comportant au moins:

. un système de réplication ou réplicon inductible ou conditionnel en amont,

. une unité amplifiable de structure -D-M-D-, les séquences D orientées en sens direct assurant l'intégration du gène M dans un gène non essentiel de ladite souche, le gène M comprenant le gène de structure de ladite protéine mis sous le contrôle d'éléments d'expression et le cas échéant de sécrétion inductibles de ladite protéine chez Bacillus, l'unité amplifiable comportant en outre éventuellement un gène sélectionnable ;

b) on effectue éventuellement une culture sur un milieu de sélection correspondant au gène sélectionnable, et

c) dans un milieu de culture approprié, on induit l'activation dudit réplicon.

Compte tenu du fait que l'amplification par activation du réplicon est suffisamment élevée, on peut en effet obtenir 40 à 80 copies du gène après une nuit d'activation du réplicon, on peut supprimer éventuellement l'étape b) de sélection sur milieu correspondant au gène sélectionnable auparavant nécessaire.

En cumulant les deux types d'amplification b) et c), on peut obtenir jusqu'à 250 copies du gène alors que le procédé par la seule pression de sélection en présence d'un gène sélectionnable donnait une amplification fournissant seulement de 20 à 30 copies du gène.

On a en outre observé que la machinerie cellulaire était toujours capable d'assurer la transcription et la traduction de toutes les séquences ainsi amplifiées ainsi que la sécrétion, le cas échéant, de la protéine produite lorsque le gène amplifié comporte également ses éléments d'expression et, le cas échéant, de sécrétion.

La présente invention a donc pour objet un procédé de production d'une protéine déterminée dans lequel l'amplification et l'activation des éléments d'expression et, le cas échéant, de sécrétion de ladite protéine ont lieu simultanément, c'est-à-dire un procédé caractérisé en ce que, à partir d'une souche du genre Bacillus dans le chromosome de laquelle ont été intégrés dans un gène non essentiel de ladite souche :

a) une unité amplifiable comportant une séquence d'ADN déterminée M encadrée par deux sequences d'ADN identiques et orientés en sens direct D, la séquence M comprenant le gène de structure de ladite protéine mis sous le contrôle d'éléments d'expression et, le cas échéant, de sécrétion inductibles chez Bacillus de ladite protéine et facultativement un gène sélectionnable, et

b) un système de réplication ou réplicon inductible ou conditionnel également intégré dans ledit gène non essentiel du chromosome de la souche en amont de l'unité amplifiable:

2

1. on sélectionne éventuellement les clones amplifiés obtenus par culture sur un milieu de sélection correspondant au gène sélectionnable et l'on prélève les souches dont le génome correspond à la présence d'un nombre de copies dudit gène accrû par rapport à la population bactérienne avant sélection ;

2. dans un milieu de culture approprié, on induit ou conditionne une amplification génique par l'activation du réplicon, et on induit l'expression et la sécrétion de la protéine par l'activation desdits éléments d'expression ;

3. on récupère ladite protéine exprimée.

Il convient de remarquer que la chronologie des étapes du procédé revêt une certaine importance dans la mesure où une étape de sélection postérieure à la suramplification de l'étape 2. par induction du réplicon conduirait à une altération de cette amplification.

On a également découvert que l'amplification peut être en outre controlée au niveau de la fonction de recombinaison homologue. En effet, lorsque le gène recE natif de Bacillus est inactivé et que son expression est bloquée, il n'y a plus de recombinaison et parallèlement l'amplification ne se produit pas malgré l'induction de l'activation du réplicon.

On peut inactiver le gène recE natif ou seulement son promoteur et complémenter par transformation avec un gène codant pour une fonction de recombinaison active, de préférence ledit gène étant placé sous le contrôle d'un promoteur inductible. Le gène codant pour la fonction de recombinaison active peut être par exemple recE de Bacillus subtillis ou recA de E. coli.

Le vecteur de transformation utilisé peut être un plasmide ou un phage lysogène comme le phage Φ 105.

Le contrôle de la fonction de recombinaison donne une second niveau de contrôle du déclenchement de l'amplification, ce double contrôle est avantageux et apporte, notamment, une sécurité dans la mesure où le contrôle de l'activation du réplicon n'est pas toujours stricte.

La présente invention a donc également pour objet un procédé de production de protéines tel que mentionné ci-dessus caractérisé en ce que le gène codant pour la fonction de recombinaison homologue dans le chromosome de ladite souche est inactivé et/ou placé sous le contrôle d'un promoteur inductible, ledit gène codant pour la fonction de recombinaison pouvant être un gène hétérologue lorsque le gène natif a été inactivé, de sorte qu'à l'étape 2 l'amplification ne se produit que si :

- on complémente le défaut de recombinaison homologue dans le chromosome de la souche par un gène codant pour la fonction de recombinaison homologue dans le chromosome de ladite souche tel que le gène recE de Bacillus subtilis ou le gène recA de E. coli, et/ou
- dans le cas où ledit gène est placé sous le contrôle d'un promoteur inductible, on induit l'activation dudit promoteur inductible de sorte que la fonction de recombinaison homologue soit exprimée dans le chromosome de ladite souche.

Selon un mode de réalisation, la présente a pour objet un procédé caractérisé en ce que le gène codant pour la fonction de recombinaison homologue est le gène natif recE de la souche de Bacillus et a été inactivé par transformation de ladite souche avec un gène recE muté, le gène recE natif étant éliminé par subustitution de gène, notamment avec l'ADN chromosomique de la souche SBEK et sélection du transformant résistant à la kanamycine.

La souche SBEK dérive de la souche SB202 de B. subtilis ; une insertion de 1.5 kb portant le gène aphA3 (35) conférant la résistance à la kanamycine a été introduit dans la séquence codante du gène recE. La transformation d'une souche de B. subtilis avec l'ADN chromosomique de la souche SBEK et la sélection des transformants résistants à la kanamycine (10 μg/ml) permet d'isoler en une seule étape des clones mutés dans le gène recE. Cette construction permet d'inactiver le gène recE résident de n'importe quelle souche de Bacillus.

La présente invention a également pour objet un procédé de préparation d'une souche de Bacillus utile dans le procédé de production d'une protéine selon l'invention dans le chromosome de laquelle un gène déterminé codant ladite protéine a été intégré, et éventuellement amplifié, caractérisé en ce que :

a) on effectue l'intégration dans le chromosome de ladite souche d'au moins une séquence d'ADN dite d'intégration comportant au moins :

. un système de réplication ou réplicon inductible ou conditionnel en amont,

. une unité amplifiable de structure -D-M-D-, les séquences D orientés en sens direct assurant l'intégration du gène M dans un gène non essentiel de ladite souche, le gène M comprenant le gène de structure de ladite protéine mis sous le contrôle d'éléments d'expression et le cas échéant de sécrétion inductibles de ladite protéine chez Bacillus, l'unité amplifiable comportant en outre éventuellement un gène sélectionnable ;

b) On sélectionne éventuellement les souches de Bacillus obtenues par culture éventuellement sur un milieu de sélection correspondant au gène sélectionnable ; et

c) on prélève les souches stables dont le génome correspond à la présence d'un nombre de copies dudit gène accrû par rapport à la population bactérienne avant sélection.

Le procédé de préparation de souche selon l'invention permet d'obtenir une souche hyperproductrice de la protéine codée par le gène hétérologue intégré qui est stable sans qu'il soit nécessaire de maintenir une pression de sélection.

Comme on l'a vu avantageusement, le gène codant pour la fonction de recombinaison homologue dans le chromosome de ladite souche est inactivé et/ou placé sous le contrôle d'un promoteur inductible pour permettre un double contrôle de l'amplification lors d'un procédé de production de protéines selon l'invention à l'aide de la souche ainsi préparée.

Mais, on a en outre découvert que l'on pouvait produire des clones de souche bactérienne de <u>Bacillus</u> portant un domaine amplifié stable lorsque l'amplification est déclenchée par activation de l'origine de réplication et ensuite stabilisée en bloquant l'expression de la fonction de recombinaison homologue.

Ce procédé permet avantageusement de préparer des clones de souche bactérienne amplifiée sans qu'aucune sélection ne soit nécessairement exercée.

La présente invention a donc également pour objet un procédé de préparation d'une souche de Bacillus utile notamment dans un procédé de production de protéines et en particulier un procédé tel que mentionné précédemment, dans le chromosome de laquelle un gène déterminé codant pour ladite protéine a été intégré et amplifié caractérisé en ce que :

a) on effectue l'intégration dans le chromosome de ladite souche d'au moins une séquence d'ADN dite d'intégration comportant au moins :

. un réplicon inductible ou conditionnel en amont,

. une unité amplifiable de structure -D-M-D-, les séquences D orientées en sens direct assurant l'intégration du gène M dans un gène non essentiel de ladite souche, le gène M comprenant le gène de structure de ladite protéine mis sous le contrôle d'éléments d'expression et le cas échéant sécrétion inductibles de ladite protéine chez Bacillus, l'unité amplifiable comportant en outre éventuellement un gène sélectionnable ;

b) on sélectionne éventuellement les souches de <u>Bacillus</u> obtenues par culture éventuellement sur un milieu de sélection correspondant au gène sélectionnable ;

c) dans un milieu de culture appropriée, on induit une amplification génétique de l'activation du réplicon ;

d) on bloque l'expression de la fonction de recombinaison homologue dans le chromosome de ladite souche ; et

e) on prélève les souches stables dont le génome correspond à la présence d'un nombre de copies dudit gène accrû par rapport à la population bactérienne avant sélection.

Le contrôle de l'expression de la fonction de reconnaissance homologue à l'étape c) se fait :

- en plaçant le gène codant pour la fonction de recombinaison homologue dans le chromosome de ladite souche sous le contrôle d'un promoteur inductible, ledit gène pouvant être le gène natif ou le gène hétérologue si le gène natif a été inactivé, auquel cas à l'étape c) on induit également l'activation dudit promoteur du gène codant pour la fonction de recombinaison et à l'étape d) on interrompt l'induction de l'activation dudit promoteur,

ou

- en transformant ladite souche avec un gène <u>recE</u> muté, le gène <u>recE</u> natif étant éliminé par substitution de gène, notamment avec l'ADN chromosomique de la souche SBEK et sélection des transformants résistants à la kanamycine.

La présente invention a également pour objet un procédé de préparation d'une protéine déterminée caractérisé en ce que l'on cultive une souche amplifiée par le procédé ci-dessus dans laquelle le gène déterminé code pour ladite protéine dans un milieu de culture et en ce que l'on prépare ensuite la protéine exprimée et le cas échéant secrétée.

Dans les procédés de préparation de souches ou de production de protéines selon l'invention, le système de réplication sera de préférence issu d'un plasmide bactérien bien que cela ne soit pas indispensable. Le système ou origine de réplication est situé au voisinage, de préférence à moins de 50 kb, de la duplication et orienté vers celle-ci. On notera que l'origine de réplication ne fait pas partie des séquences amplifiées.

A titre de réplicon conditionnel ou inductible, on pourra utiliser un réplicon thermosensible tel que le plasmide pE194 dont la réplication est naturellement thermosensible active à 37°C et inactivée à 51°C.

L'unité amplifiable sera comprise de séquences non réplicatives chez <u>Bacillus</u>, de préférence issues d'un plasmide bactérien bien que cela ne soit pas non plus indispensable. En particulier, les séquences D peuvent provenir du plasmide pBR322.

De façon générale, le gène sélectionnable sera un gène de résistance à un composé chimique, en particulier un antibiotique : kanamycine, ou chloramphénicol, par exemple.

Dans ces conditions, la sélection de la souche est aisée puisqu'il suffit de sélectionner une souche très résistante à l'antibiotique.

Grâce au procédé selon l'invention, on peut obtenir une souche hyperproductrice de la protéine codée par le gène hétérologue intégré, qui est stable sans qu'il soit nécessaire de maintenir une pression de sélection.

On peut en particulier citer comme espèces de bactéries du gène Bacillus utilisées selon l'invention :

- Bacillus subtilis
- Bacillus alcalophilus
- Bacillus brevis
- Bacillus circulans
- Bacillus lentus
- Bacillus licheniformis
- Bacillus macerans
- Bacillus megaterium
- Bacillus polymyxa
- Bacillus pumilus
- Bacillus stearothermophilus
- Bacillus coagulans

Des vecteurs intégratifs ont été construits dans le but d'intégrer des gènes étrangers dans le chromosome de Bacillus. Ces vecteurs se caractérisent essentiellement par l'existence de segments homologues du chromosome de Bacillus qui assure l'intégration. Sur le plan technique, ces vecteurs sont connus dans leurs principes et leurs réalisations.

En particulier notamment dans FR 84 06701 (2 563 533), il est nécessaire pour que l'intégration puisse se faire que le vecteur présente une séquence d'ADN identique à celle du chromosome de la souche de Bacillus. Dans certains cas, on utilise des vecteurs portant des fragments du chromosome de Bacillus sauvage, par exemple tout ou partie du gène thy B, ou d'un gène qui sera nommé X.

On peut également intégrer d'abord dans le chromosome une séquence déterminée provenant, par exemple, d'un plasmide bactérien tel que pBR322, dans ce cas il suffit ensuite d'utiliser un vecteur d'intégration portant la même séquence.

Dans le cas de Bacillus subtilis, le système de réplication et l'unité amplifiable -D-M-D- seront avantageusement intégrés entre les séquences thyB et X de Bacillus subtilis à la place du gène ilvA de ladite souche.

Dans un mode de réalisation particulier des procédés selon l'invention, les éléments d'expression et de sécrétion du gène codant pour ladite protéine consistent dans le promoteur du gène sacB inductible par le saccharose accompagné de la région sacR .

On utilisera de préférence la partie du locus sacRB de Bacillus subtilis qui contient

1) le locus sacB contenant le promoteur et la région de régulation du gène sacB, et

2) la région de sacB codant la séquence signal de la lévane-saccharase.

En particulier, on a intégré le gène celA de clostridium thermocellum codant pour l'endoglucanase A. L'endoglucanase A a été choisie comme modèle de protéine pour tester l'efficacité du système. Cette protéine a été choisie compte tenu de sa taille et de son activité enzymatique qui sont facilement détectables.

De préférence, on utilisera une souche de Bacillus subtilis présentant la mutation sacU$^h$ favorisant la production de lévane-saccharase.

On a observé que l'expression des gènes amplifiés et la sécrétion de la protéine est plus importante lorsque la culture arrive en phase stationnaire, ce jusqu'à 24 heures après induction de l'amplification génique à 37°C.

Ainsi, le gène celA mis sous le contrôle de signaux d'expression et de sécrétion du "système sacB" chez Bacillus subtilis aboutit à une hyperproduction de l'EGA après induction de l'expression par le saccharose et ceci à un niveau particulièrement élevé dans un contexte génétique sacU$^h$. Après induction de l'amplification génique par réplication de pE194 et induction de l'expression de celA par le saccharose, le surnageant des cultures de Bacillus subtilis ainsi obtenu contenait le produit du gène celA, protéine dite endoglucanase A, cette protéine étant largement majoritaire dans le surnageant (plus de 90 % des protéines). Sa concentration a été évaluée à environ 10 mg/l par unité de densité optique, soit environ 10 fois supérieur au rendement obtenu lorsque le clonage de celA était effectué sur plasmide multicopie tel que pC194. L'analyse de la séquence NH$_2$-terminale de la protéine a montré que celle-ci était correctement maturée lors de sa sécrétion. Enfin, un test d'activité enzymatique a permis de montrer que la protéine sécrétée était active.

Un gène différent de celA a également pu être amplifié selon le même procédé. Il s'agit de CAT. Son produit, la chloramphenical acétyl transferase (CAT) est intracellulaire. Une augmentation de 90 fois de CAT dans les extraits bruts a été observée après suramplification (de 12 U/mg à 900 U/mg). Le procédé permet donc de surproduire des protéines intracellulaires aussi efficacement que des protéines sécrétées dans le surnageant.

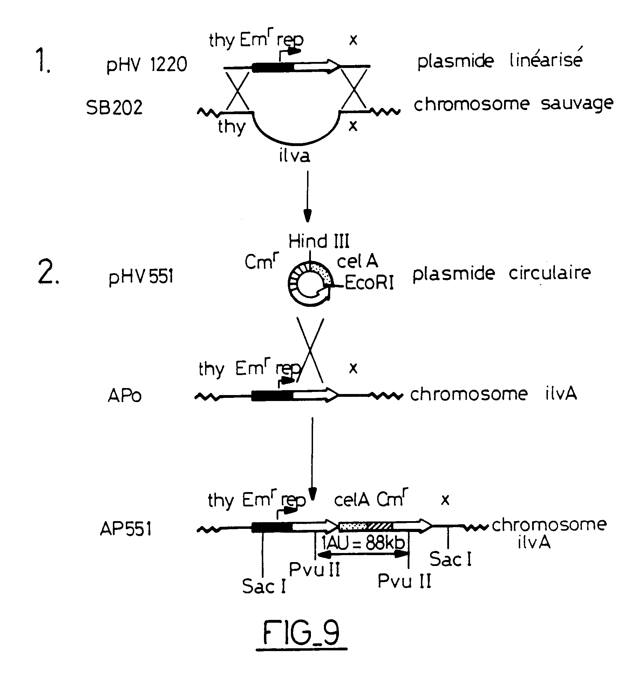

La présente invention a également pour objet les souches de Bacillus, en particulier de Bacillus subtilis obtenues par la mise en oeuvre du procédé de préparation de souche selon l'invention, en particulier la souche AP551 telle que représentée à la figure 9.

D'autres caractéristiques de la présente invention apparaîtront à la lumière des exemples qui vont suivre faits en référence à des figures.

Figures

La figure 1 représente le tableau 1 de l'exemple 1 dans lequel sont énumérés les souches et plasmides utilisés.

La figure 2 représente le tableau présentant les résultats d'activité CMC-ase dans des surnageants de culture de différentes souches transformées de Bacillus subtilis (exemple 1).

La figure 3 représente la construction de pGJ1. Les lignes en caractère gras représentent les fragments d'ADN de pBS430. Les rectangles hachurés représentent les régions codant le gène celA. Les rectangles blancs représentent les régions de pCT105 flanquant le gène celA. Les flèches indiquent le sens de transcription des gènes.

La figure 4 représente l'expression du gène celA du plasmide pGJ2 dans différentes souches de Bacillus subtilis. La courbe en caractère gras représente la croissance bactérienne. Les symboles correspondent à l'activité spécifique CMC-ase quand les souches hôtes étaient : o, sacU$^h$ ; Δ, sacS$^h$ ; □, sacS$^c$ ; ◊, type sauvage. L'activité spécifique a été calculée comme unité d'activité CMC-ase par mg de protéine. La concentration de protéine a été corrélée à l'absorbance de la culture cellulaire, une unité de densité optique à 660 nm correspondant à 250 μg de protéine bactérienne par ml.

La figure 5 représente la fusion de la région codant la séquence mature de l'EGA à la région codant la séquence signal de la Lvs par mutagénèse dirigée. DGF3 est l'amorce mutagénique. La séquence délétée de PGJ3 (figure 6) est présentée en épingle à cheveux.

La figure 6 représente la construction de pGJ3. Les symboles utilisés sont comme dans la figure 1. DGF3 correspond à l'amorce mutagénique et SEL1 à l'amorce de sélection.

La figure 7 représente l'électophorèse sur gel de polyacrylamide SDS. Les aliquotes (25 μl) du surnageant de Bacillus subtilis QB136 après culture en phase logarithmique, concentrés x 25 sur une membrane Amicon, ont été appliqués sur un gel de polyacrylamide 10 %. Sur la figure sont représentés des tracés de densitomètre de gel coloré au bleu de Coomassie : a, QB136 b, QB136 (pGJ2) ; c, des marqueurs de référence de poids moléculaire, phosphorylase b (94 kdal), la sérum albumine bovine (67 kdal), la lévane-saccharase (50 kdal), l'ovalbumine (43 kdal) et l'anhydrase carbonique (30 kdal).

La figure 8 représente le tableau 3 de l'exemple 2 énumérant les souches et plasmides utilisés.

a : des modifications génétiques dues à l'insertion de plasmides dans le chromosome de Bacillus subtilis sont les suivantes :

(1) ins suivi par un "plasmide" représente le plasmide inséré.

(2) del ou dup suivi du "gène ou de la séquence" représentent des délétions ou duplications respectivement, résultant de l'insertion du plasmide.

(3) pBR322Δ 81 représente des séquences de pBR322 présentes dans pHV33Δ81. Le phénotype sacU$^h$ conduit à l'hyperproduction de Lvs. CMC représente la faculté d'une souche à hydrolyser la carboxyméthylcellulose.

La figure 9 représente la construction des souches AP0 et AP551. Les symboles utilisés sont :

▥ :gène de résistance au chloramphénicol Cm$^r$

▦ :gène celA de fusion contenant le promoteur du gène de la lévane- saccharase de Bacillus subtilis et la région codant sa séquence signal fusionnée en phase de lecture avec la séquence codant la séquence mature de l'endoglucanase A de C. thermocellum

⇨ : séquences pBR322 de 3,9 kbp

■ : plasmide pE194 portant le gène de résistance à l'érythromycine Emr, la flèche indique le sens de réplication. Les lignes représentent les séquences chromosomiques adjacentes : X et thy se réfèrent à un segment chromosomique de contenu génétique indéterminé, et le gène chromosomique thyB, respectivement. L'unité d'amplification (AU) est indiquée. Les sites de restriction PvuIII et SacI utilisés pour caractériser les structures amplifiées sont indiqués.

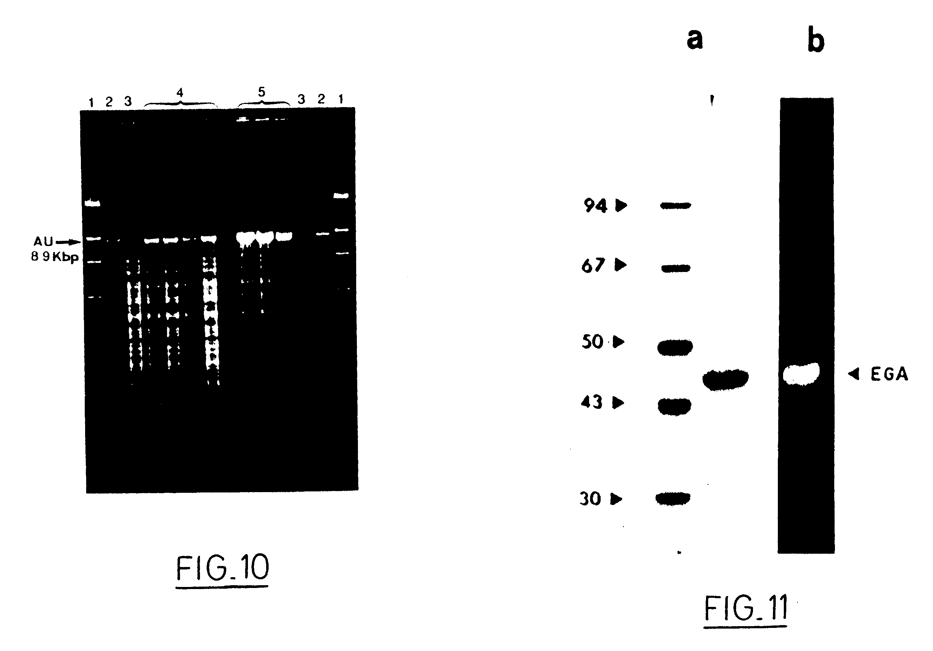

La figure 10 représente l'amplification génique dans AP551 et AP551i. L'ADN chromosomique a été extrait de 4 clones sélectionnés sur 50 μg/ml de chloramphénicol à partir de AP551i (lignes 4), et de 3 clones sélectionnés de la même façon à partir de AP551, puis cultivés une nuit à 37°C (lignes 5). Après restriction par PvuII et séparation par électrophorèse, une bande fluorescente de 8.9 kb est révélée dans ces échantillons. Elle est absente de l'ADN chromosomique de AP551 avant amplification (ligne 3) et correspond à la taille de l'U.A. (pHV551 linéarisé, ligne 2). Ligne : lambda HindIII.

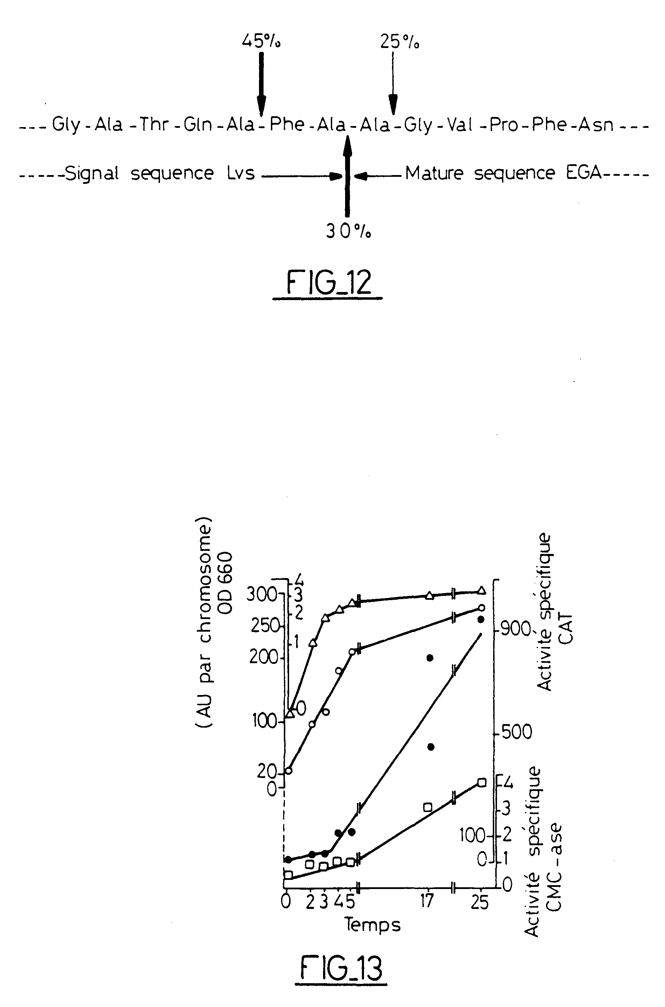

La figure 11 représente un SDS-PAGE suivi par une coloration pour l'activité cellulase. Des aliquots (20 μl) de surnageants de la phase stationnaire de la souche sacU[h] AP551 de Bacillus subtilis induite, concentrés 20 fois sur une membrane Amicon, ont été appliqués sur une gel de polyacrylamide 10 %. La colonne a, protéine à droite d'un jeu de marqueurs de masse moléculaire : phosphorylase b (94 kdal), sérum albumine bovine (67 kdal), lévane-saccharase (50 kdal), ovalbumine (43 kdal), anhydrase carbonique (30 kdal). Colonne b, activité selon Béguin[52].

La figure 12 représente les données concernant l'extrémité NH$_2$-terminale de l'EGA. Les flèches indiquent les sites de clivage dans la protéine sécrétée dans Bacillus subtilis sacU[h] AP551. Le pourcentage de chaque produit est indiqué.

La figure 13 représente la relation cinétique entre l'amplification génique et l'expression. Les différents symboles utilisés sont

Δ densité optique

○ nombre de copies de l'unité d'amplification

● activité spécifique CAT

□ activité spécifique CMC-ase.

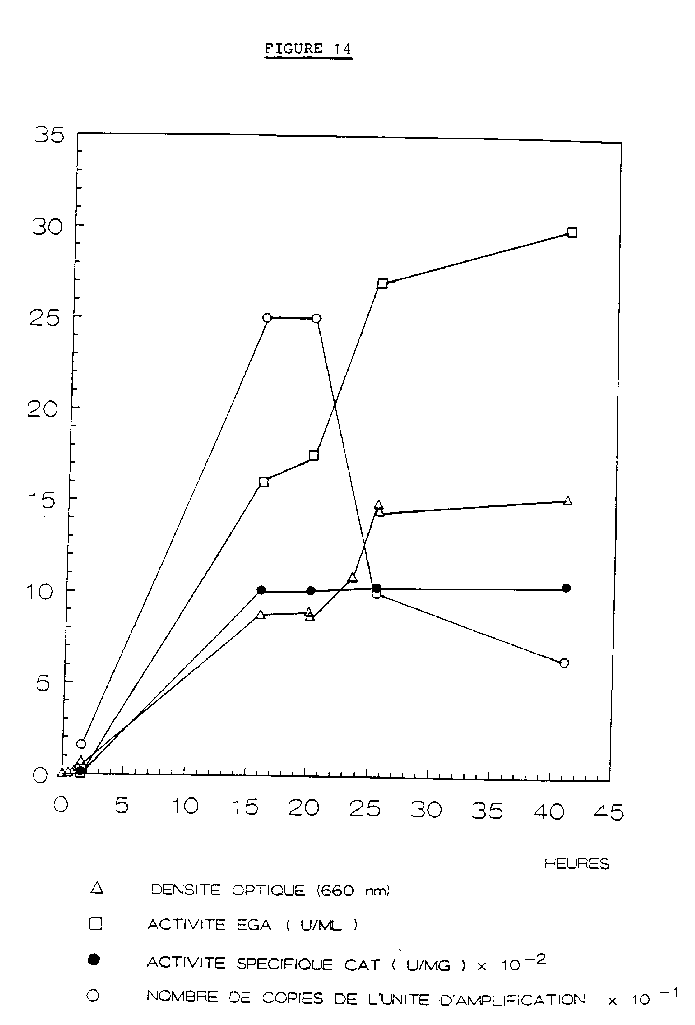

La figure 14 représente la production d'EGA et de CAT en fermenteur de 20 litres,

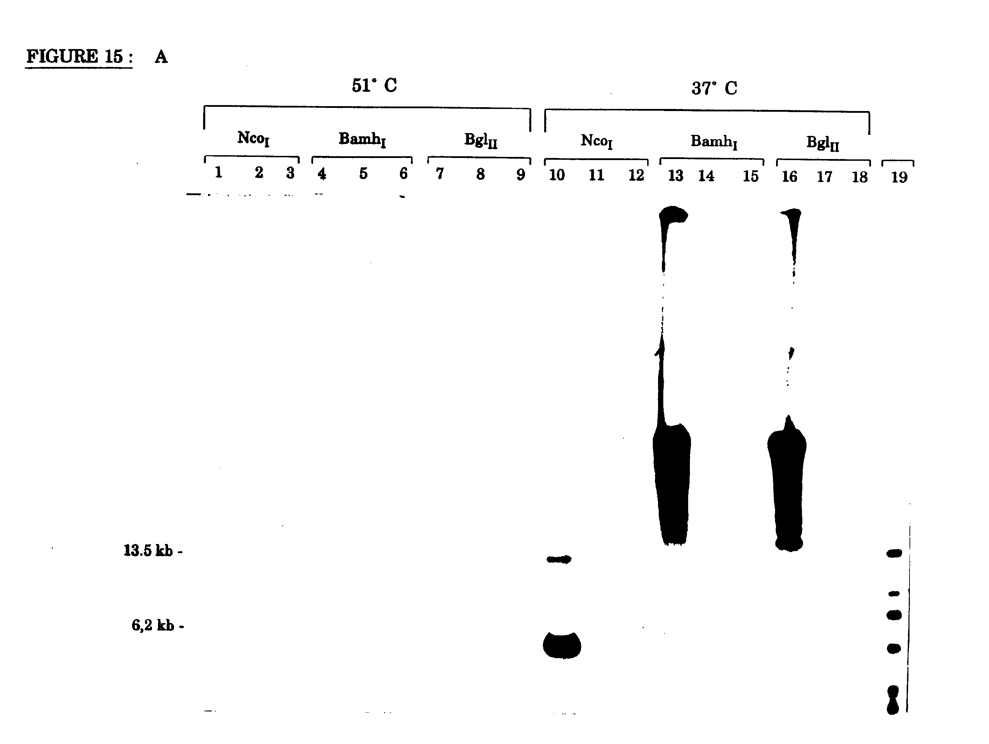

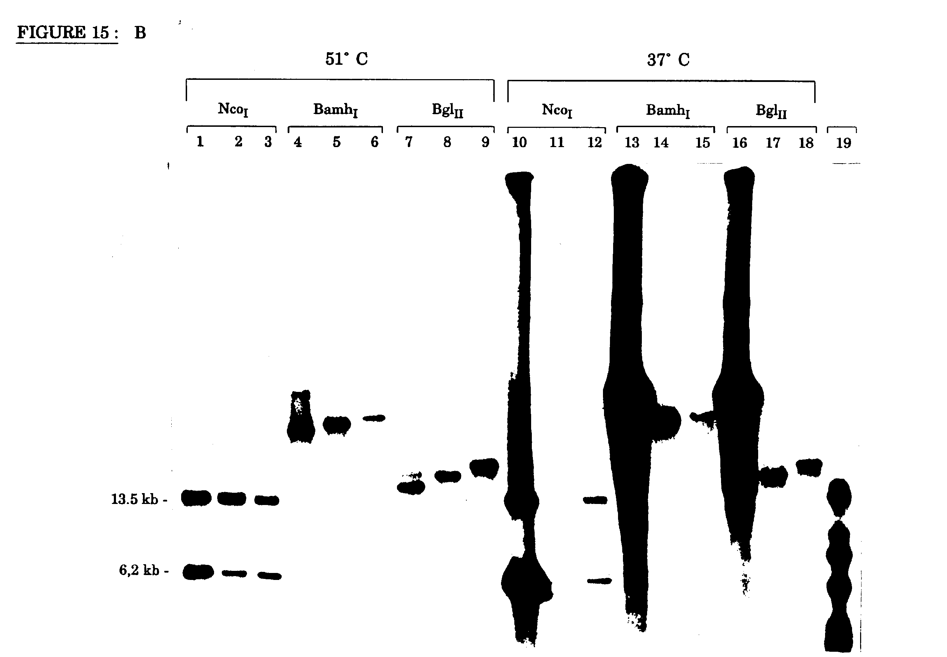

Les figures 15A et 15B représentent les résultats de l'amplification de l'exemple 4.

LEGENDE FIGURE 15 :

- A : 24 hrs exposition
- B : 48 hrs exposition

Distribution des puits:

● ADNs extraits des souches cultivées à 51°C
  - puits 1, 4, 7 : souche AP2
  - puits 2, 5, 8 : souche AP rec 4
  - puits 3, 6, 9 : souche AP rec 18

● ADNs extraits des souches cultivées 2 hrs à 37°C :
  - puits 10, 13, 16 : souche AP2
  - puits 11, 14, 17 : souche AP rec 4
  - puits 12, 15, 18 : souche AP rec 18

Puits 19 :        marqueur de taille Raoul I

                  poids moléculaires des plus grands fragments (kb) 19 ; 15,7 ; 9,1 ; 8,0 ; 5,9 ; 4,6

## EXEMPLE 1 : Sécrétion inductible de cellulase de Clostridium thermocellum dans Bacillus subtilis sans amplification

Un système hôte-vecteur pour la sécrétion inductible pendant la phase logarithmique de croissance dans Bacillus subtilis a été développé. Le promoteur du gène de la lévane-saccharase de Bacillus subtilis et la région codant sa séquence signal ont été utilisés. L'endoglucanase A de Clostridium thermocellum a été utilisée comme modèle de protéine pour tester l'efficacité du système. Une sécrétion inductible efficace d'endoglucanase A a été observée en utilisant soit la séquence signal de la lévane-saccharase, soit sa propre séquence signal.

Divers protéines hétérologues ont été sécrétées dans Bacillus subtilis et la plupart des vecteurs de sécrétion développés utilisait le système de sécrétion de l'alpha amylase [28, 29,31] bien que plus récemment des systèmes d'exportation de protéases aient été utilisés [14, 15, 29, 36, 38]. Toutefois, l'expression à partir de ces systèmes intervient pendant la phase stationnaire quand la plupart des enzymes sécrétés de Bacillus subtilis y compris les protéases majoritaires sont sécrétés. Ici, est décrit un vecteur de secretion basé sur le système de la lévane-saccharase de Bacillus subtilis qui est actif pendant la phase de croissance logarithmique. La lévane-saccharase est codée par le gène sacB et exprimée à partir d'un promoteur fort inductible par le saccharose qui est situé dans le locus sacR à proximité. Le contrôle de son expression a été abondamment étudié [12, 34] et plusieurs mutants de régulation sont disponibles : sacQ[h], sacS[h], sacU[h] [20, 21].

L'endoglucanase A extracellulaire (EGA ; 1,4-bêta-D-glucan glucanohydrolase EC 3.2.1.4) de la bactérie thermophile anaérobie Clostridium thermocellum codée par le gène celA a été utilisée comme modèle de protéine pour tester l'efficacité du système de sécrétion sacB dans Bacillus subtilis. Cette protéine a été choisie pour sa taille qui est approprié [23] et son activité enzymatique qui est facilement détectable directement sur boîte de Petri [10]. En outre l'EGA a été caractérisée et purifiée [23,32] et la séquence d'ADN [5] de son gène et les sites d'inition de la transcription [7] sont connus. Le poids moléculaire calculé de la séquence mature de l'EGA est de 49 125, mais sur gel de polyacrylamide SDS de surnageants de culture de C. thermocellum, il apparaît une bande de 56 000 qui est attribuée à une glycosylation [5]. Ces facteurs facilitent l'utilisation de cette protéine comme modèle pour la sécrétion hétérologue dans Bacillus subtilis.

On a montré que Bacillus subtilis peut sécréter l'EGA active enzymatiquement dans le milieu de culture après transformation avec des plasmides hybrides après induction par le saccharose. On compare également l'efficacité des séquences signal de la lévane-saccharase et de l'EGA lorsqu'elles sont fusionnées avec le gène de structure de l'EGA.

## 1. Matériel et méthodes

### 1.1 Souches, phage, plasmides et milieux

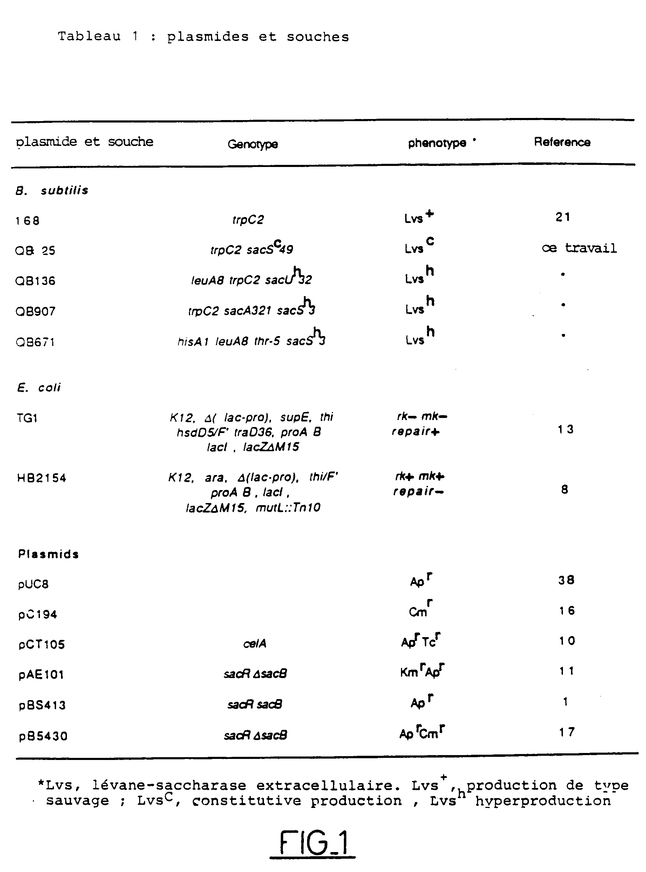

Les souches et plasmides utilisés sont énumérés dans le tableau 1 de la figure 1. Le phage M13mp18-am4 a été utilisé pour la mutagenèse dirigée. Pour la croissance d'E.coli, un milieu nutritif Luria [22] a été utilisé supplémenté avec l'ampicilline (Ap) (100 µg/ml) du chloramphénicol (Cm) (10 µg/ml), de la kanamycine (Km) (50 µg/ml) ou de la tétracycline (Tc) (10 µg/ml). Pour la croissance de Bacillus subtilis, un milieu nutritif de sporulation [30] a été utilisé supplémenté avec Cm (5 µg/ml) ou Km (5 µg/ml) pour la sélection et 2 % (w/v) de saccharose pour l'induction du promoteur de sacB. La transformation de E. coli a été effectuée selon Cohen et al. [9] et la transformation de Bacillus subtilis a été effectuée tel que décrit par Anagnostopoulos et Spizizen[2].

### 1.2 Activité endoglucanase A

La carboxyméthylcellulose (CMC) (N° C-4888 milieu visqueux, Sigma CO) a été utilisée comme substrat pour l'EGA. La détection de l'activité endoglucanase par le dosage au rouge Congo sur boîte de Petri, a été effectuée tel que décrit par Cornet et al.[10]. Tout dosage d'enzyme a été effectué dans un tampon PC (50 mM $K_2HPO_4$, 12 mM d'acide citrique, pH 6,3) à 60°C. L'activité carboxyméthylcellulase (CMC-ase) a été mesurée selon Béguin et al.[6]

Une unité d'activité a été définie comme la quantité d'enzyme qui libérait 1 µmole d'équivalent glucose par minute. La détection d'activité cellulase dans des gels de polyacrylamide en utilisant le rouge Congo a été effectuée selon Béguin[4].

### 1.3 Mutagénèse dirigé

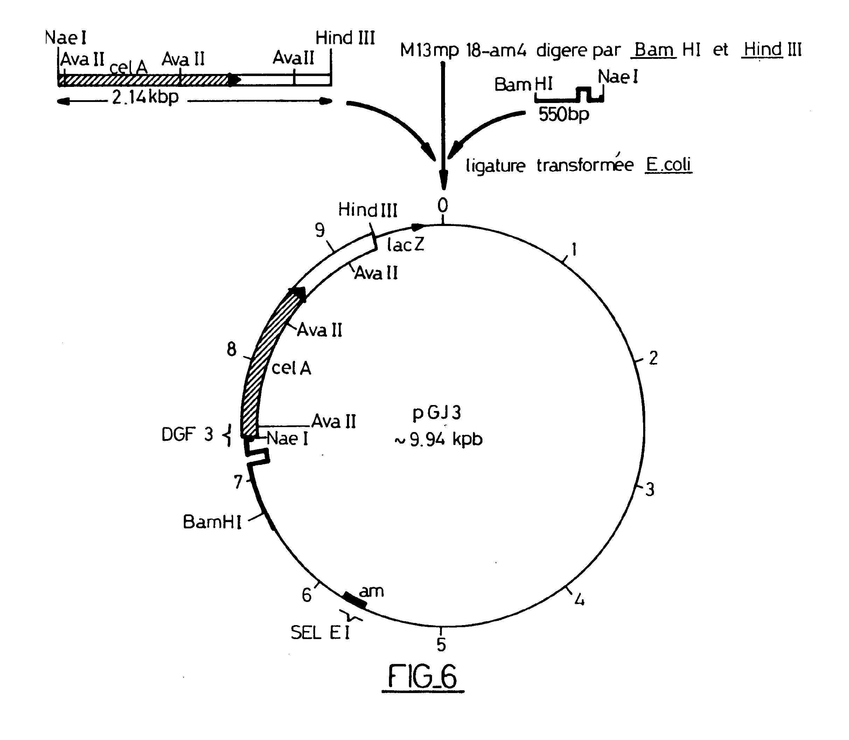

La procédure utilisée a été décrite par Carter et al.[8] PGJ3 (figure 6) a été utilisé pour produire une matrice monobrin qui a été amorcée avec une amorce mutagénique DGF3 (5'AAAAGGCACACCTGCGGCAAACGC-TTGAGT 3') et une amorce de sélection SEL1 (5' AAGGAGTCTGTCCATCAC 3') utilisée pour reverser la mutation "ambre" présente dans un des gènes essentiels du phage M13 mp18-am4. La transfection dans des cellules HB2154 qui sont déficientes dans leur système de réparation permet la récupération du simple brin possédant la mutation introduite par DGF3. Les 15 nucléotides 5' de l'amorce mutagénique DGF3 sont complémentaires des premiers 15 nucléotides de la séquence mature de EGA et les 15 nucléotides 3' sont complémentaires des 15 derniers nucléotides de la séquence signal de la lévane-saccharase (Lvs) contenue dans pAE101[11]. Des mutants contenant la fusion en phase de lecture entre la séquence signal de la Lvs et la séquence nature de EGA ont été hybridés fortement à DGF3 après un lavage stringent. La mutation correspondant à une délétion des 19 nucléotides de la séquence signal de EGA a été vérifiée par séquençage au didésoxynucléotide[27] utilisant l'amorce synthétique DGF4 (5' TCGGCAATAGAAGTAGG 3') qui s'hybride en aval de la région délétée.

### 1.4 Construction de pAE119, un plasmide pour la délétion de la région sacRB dans Bacillus subtilis

La région sacRB a été délétée par recombinaison. Le plasmide pBS413[1] contient la région sacRB sur des fragments HindIII contigus. Le plasmide a été linéarisé avec ClaI qui coupe dans le gène sacB et a été traité avec l'exonucléase Bal31 pour déléter la région sacRB complète. Le fragment a ensuite été ligaturé au gène aphA3 de Streptococcus faecalis pour donner pAE119 qui n'est pas réplicatif dans Bacillus subtilis. Le gène aphA3 utilisé est inclus dans un fragment ClaI de 1,5 kbp de pAT21[35].

## 2. Résultats

### 2.1 Expression du gène celA dans Bacillus subtilis sous le contrôle de la région sacR

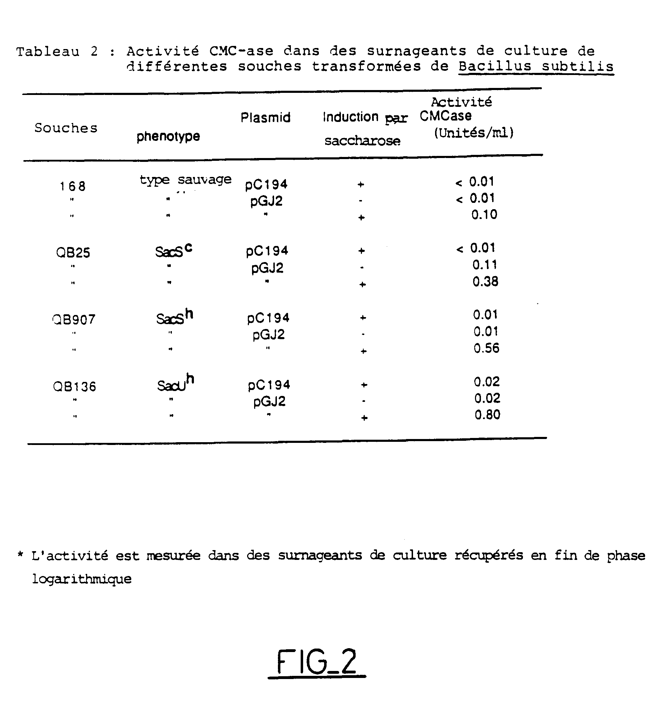

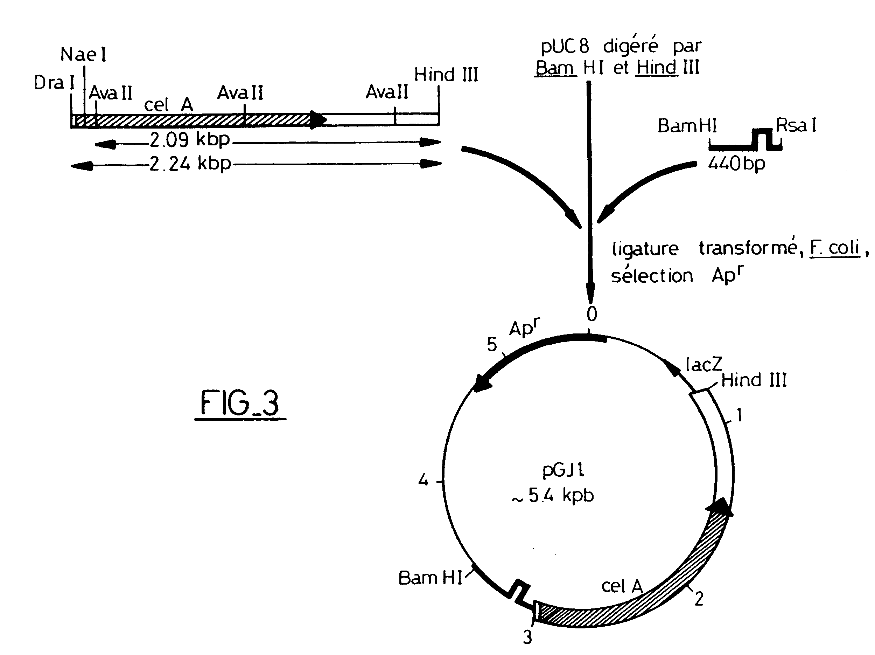

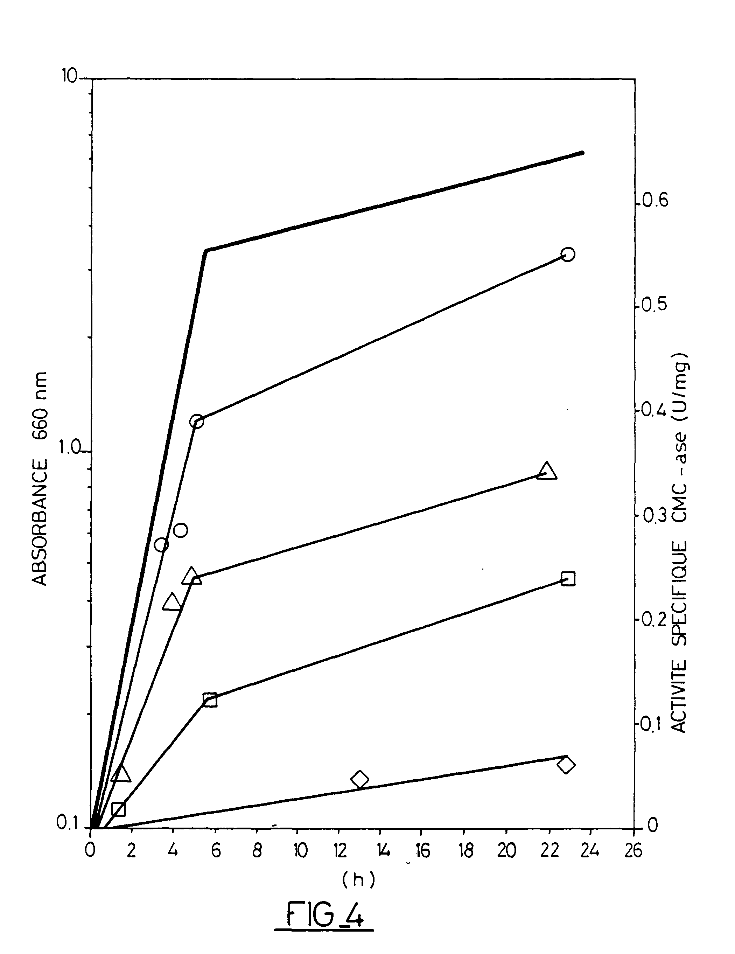

Une construction pour l'expression de l'EGA a été faite en procédant à une ligature à trois fragments entre (a) le vecteur pUC8 coupé avec BamHI et HindIII (b) BamHI-Rsal de 440 bp de la région sacR contenant le promoteur de sacB et sa région de régulation issue de pBS4 30[3,7] et (c) un fragment Dral-HindIII de 2,2 kbp du plasmide pCT105[10] contenant le site de fixation de ribosomes, la région codant EGA (séquence signal et séquence mature[5]) et le site de terminaison du gène[7] (figure 3). La construction recherchée pGJ1 a été isolée sous forme d'un transformant Ap$^r$ de E. coli présentant le phénotype CMC$^+$ dans le test sur boîte de Petri [10]. Pour tester la sécrétion de l'EGA dans Bacillus subtilis, pC 194[16] un plasmide de 2,91 bp ayant une origine de réplication fonctionnelle de Bacillus subtilis et un gène de résistance à un antibiotique (Cm) a été ligaturé à pGJ1 au site HindIII. Le plasmide résultant pGJ2 a été introduit dans différentes souches de Bacillus subtilis : 168 (type sauvage), sacS$^c$, sacS$^h$ et sacU$^h$ (figure 1). Les transformants Cm$^r$ de Bacillus subtilis portant le gène celA de C. thermocellum sur le plasmide multicopie pC194 ont été identifiés par des zones élargies de l'hydrolyse de CMC après induction par le saccharose. La taille des halos toutefois varie, dépendant de la souche hôte. Les dosages de l'endoglucanase des surnageants de culture liquides ont montré une sécrétion d'EGA inductible par le saccharose (figure 4, figure 2). Ces résultats indiquent que la régulation de l'expression du gène celA est la même que celle du gène sacB. Aucune expression du gène celA n'a été détectée en l'absence de saccharose dans les souches inductibles. Le plus haut niveau de sécrétion de EGA a été obtenu avec le mutant sacU$^h$ qui hypersécrètent aussi la lévane-saccharase en produisant environ 100 fois plus de cette enzyme que des cellules du type sauvage. La sécrétion d'EGA dans une souche sacU$^h$ est seulement environ 10 fois supérieure que dans une souche sauvage hébergeant la même construction. Cependant l'expression du gène provient d'un plasmide multicopie. Or l'expression dans une souche sacU$^h$ de sacB sur un plasmide multicopie est seulement 5 à 10 fois plus forte que le niveau obtenu dans un mutant sacU$^+$. Bacillus subtilis possède une activité bêta-1,4-endoglucanase endogène[25]. Toutefois, dans une souche de Bacillus subtilis sacU$^h$ (pGJ2) qui présente une activité endogène plus importante que la souche de type sauvage, cette activité représente seulement environ 2 % de l'activié CMC-ase.

Les dosage d'endoglucanase ont montré que les extraits cellulaires de souche sacU$^h$ (pGJ2) contiennent environ 20 % de l'activité EGA totale indiquant que la plupart de l'EGA était sécrétée dans le milieu (80 %).

### 2.2 Sécrétion de l'EGA par la séquence signal de la lévane-saccharase et la région de régulation sacR

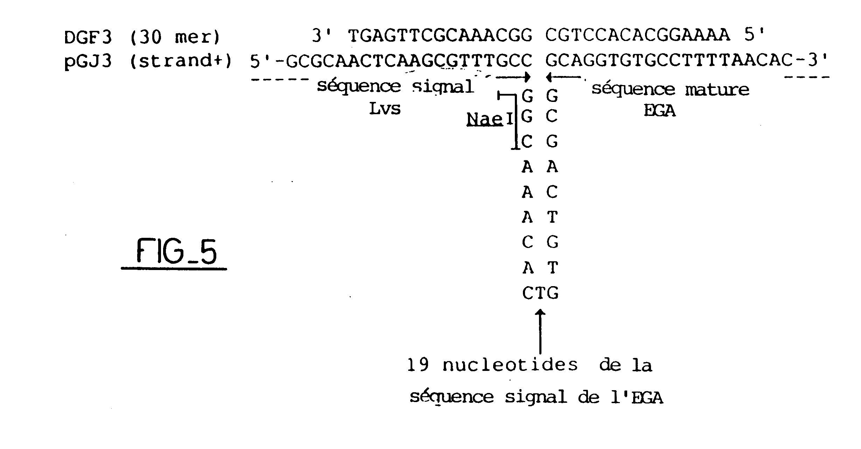

La région de l'ADN codant la séquence signal de la lévane-saccharase et portant sacR a été fusionnée à la région codant la séquence mature de l'EGA par mutagénèse dirigée dans le vecteur M13[8] (figure 5). Le vecteur M13mp18-am4[8] qui a une mutation "ambre" dans un gène essentiel du phage a été coupé avec BamHI et HindIII et ligaturé à (a) un fragment BamHI-Nael de 0,55 kbp du plasmide pAE101[11] portant sacR et la séquence signal de la lévanesaccharase (b) un fragment Nael-HindIII de 2,14 kbp du plasmide pCT105[10] contenant l'ADN codant la séquence mature EGA et 19 nucléotides de la séquence signal de EGA (figure 6). Le plasmide résultant a été dénommé pGJ3. Les 19 nucléotides appartenant à la séquence signal de EGA ont été délétés de pGJ3 et des constructions contenant la séquence recherchée ont été dénommés pGJ4. Toutefois les plasmides contenaient une insertion d'environ 0,9 kbp dans M13mp18 à la place des 2,7 kbp attendus, correspondant à une délétion d'environ 1,8 kbp dans la région 3' de l'insert. Ceci a été vérifié par séquençage avec l'amorce universelle. Le gène de fusion complet a été reconstruit. Trois fragments (a) 0,58 kbp du fragment BamHI-Avall de pGJ4 contenant la fusion en phase avec la séquence signal de la Lvs et de la séquence mature de EGA (b) le fragment Avall-HindIII partiel de 2,09 kbp de pCT105 (figure 3) et (c) le vecteur pUC8 coupé avec BamHI-HindIII, ont été ligaturés ensemble. Le plasmide résultant contenant la fusion sacB-celA a été dénommé pGJ5.

Pour tester la sécrétion par Bacillus subtilis de EGA par cette fusion, le plasmide pC194 a été ligaturé au site HindIII de pGJ5. Le plasmide résultant pGJ6 a été introduit dans Bacillus subtilis sacU$^h$ (QB 136). Tous les transformants Cm$^r$ testés possédaient un phénotype CMC$^+$ inductible par le saccharose. Le surnageant de 1 clone a été dosé quant à son activité sur CMC après induction par le saccharose, et le niveau d'activité dans le surnageant était le même que celui de QB 136 (pGJ2). Ces résultats indiquent que les séquences signal de la Lvs et l'EGA sont également efficaces pour la sécrétion dans Bacillus subtilis.

### 2.3 Poids moléculaire et stabilité de l'EGA sécrétée par Bacillus subtilis

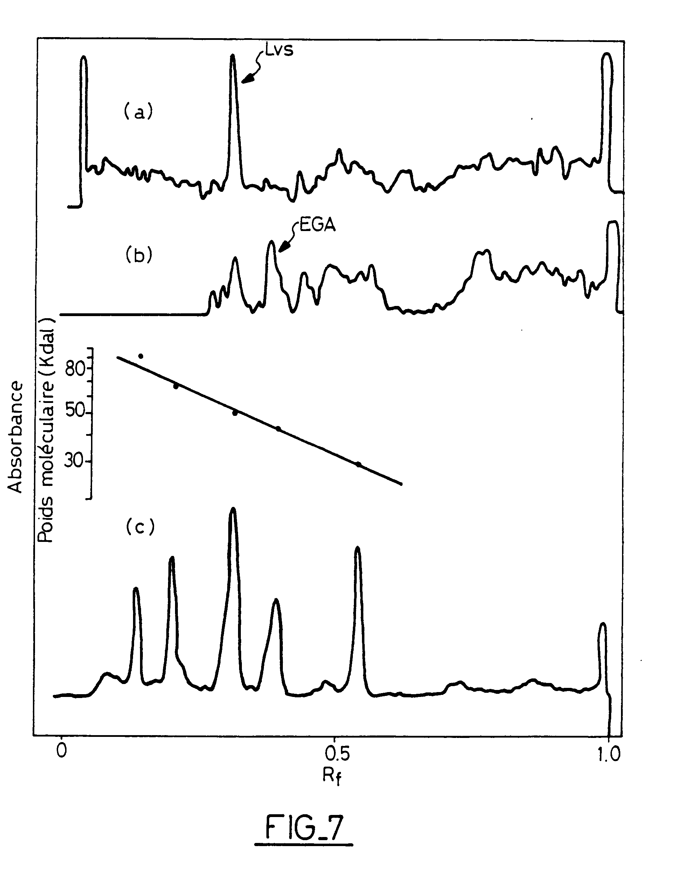

L'analyse des surnageants de Bacillus subtilis QB 136 induit par le saccharose portant pGJ2 ou pGJ6 par gel de polyacrylamide SDS a révélé une bande de protéine avec une masse moléculaire de 46 000 qui n'était pas détectée dans les contrôles (figure 7). Cette bande possède une activité CMC-ase par transfert sur gel d'agarose contenant de la CMC[4]. La masse moléculaire de la protéine correspondant à cette bande est compatible avec la masse moléculaire calculée de la séquence mature EGA, 49 125, tel que l'on peut la déduire de la séquence nucléotidique[5]. Les bouches QB136 et QB136(pGJ2) ont été induites avec du saccharose et des surnageants ont été dosés pour leur activité Lvs. Les souches non transformées produisent trois fois plus d'activité Lvs que les souches portant pGJ2. Ce résultat est en accord avec les courbes de densitomètre des gels de polyacrylamide au bleu de Coomassie (figure 7) et semble indiquer une compétition entre le gene sacB et le gène hétérologue sacB/celA pour l'expression ou la sécrétion (ou les deux).

### 2.4 Expression du gène de fusion sacB-celA dans un contexte délété en sacRB

Pour tester si l'expression de sacB à partir du gène chromosomique interfère avec une surproduction de celA, la région sacRB a été délétée du chromosome des souches hôtes. Pour ce faire, les souches hôtes ont été transformées avec pAE119 (voir matériels et méthodes) et des tranformants Km[r] ont été sélectionnés. Dans plus de 98% des transformants, la région sacRB a été remplacée par aphA3 par un double crossing over. Le plasmide pGJ2 a été introduit dans Bacillus subtilis QB671 dans lequel la région sacRB avait été délétée tel que décrit ci-dessus. QB671 est isogénique avec QB907[20], excepté qu'elle est sacA[+] (le saccharose inhibe la croissance dans les souches sacA[-]-sacB[-]). Les souches QB671 (pGJ2) et QB671 $\Delta$ sacRB (pGJ2) ont été mises en croissance dans des milieux appropriés et induites avec le saccharose. Les activités CMC-ase des surnageant ont été dosées et il n'a pas été possible des les distinguer.

### 2.5 Conclusion

La protéine hétérologue EGA de C. thermocellum a été exprimée et sécrétée à partir de Bacillus subtilis en utilisant le promoteur de sacB après induction par le saccharose. La sécrétion de EGA intervient durant la phase de croissance logarithmique et s'accumule dans le milieu. la concentration en EGA est plus grande durant la phase stationnaire indiquant que la protéine est sécrétée sous une forme stable. Une sécrétion a été obtenue en utilisant soit la séquence signal de Lvs, soit sa propre séquence signal. L'analyse de ces deux séquences signal n'a montré aucune différence significative par rapport à des séquences signal typiques de bactéries gram positives. L'expression de l'enzyme dans différents environnements génétiques de Bacillus subtilis (sacS[c], sacS[h], sacU[h]) a montré que sa régulation était comme celle du gène sacB et on retrouve une forte activité enzymatique dans le surnageant de culture d'une souche sacU[h] de Bacillus subtilis. La taille de la protéine (environ 46 kilodaltons) est compatible avec le poids moléculaire déduit de la séquence de la protéine mature.

Le niveau de sécrétion de Lvs diminue quand le gène celA est exprimé à partir du promoteur sacB sur vecteur multicopie pC 194 semblant indiquer une compétition entre le système homologue et le système hétérologue pour l'expression ou la sécrétion (ou les deux). Toutefois dans une souche $\Delta$sacRB, le niveau de EGA sécrété n'est pas augmenté. On peut présumer en conséquence que la présence d'une unique copie du gène sacB sur le chromosome n'entre pas en compétition de manière significative avec l'expression de gène à partir du promoteur de sacB sur un plasmide multicopie.

L'activité EGA dans le surnageant d'une souche sacU[h] était de 0,8 U/ml. Comme l'activité spécifique d'EGA purifiée est de 140 U/mg[24] ceci correspond à une concentration de protéine d'environ 1 mg/l/unité $DO_{660}$. Cette valeur se compare avantageusement avec celle obtenue pour la production de EGA dans C. thermocellum et E. coli[10].

### EXEMPLE 2: Hypersécrétion d'une cellulase de Clostridium thermocellum dans Bacillus subtilis par induction de l'amplification de l'ADN chromosomique

### 1. Résumé

Le fragment d'ADN contenant le promoteur du gène de la lévane-saccharase (Lvs) de Bacillus subtilis et la région codant sa séquence signal, fusionné en phase avec la séquence codant la séquence mature de l'endoglucanase A de Clostridium thermocellum a été introduit et amplifié à un site spécifique du chromosome de Bacillus subtilis. L'amplification d'ADN induite par la réplication du plasmide pE194 thermosensible a permis d'augmenter le nombre de copies du gène jusqu'à 250 copies par chromosome. Cette amplification d'ADN a conduit à la synthèse et à la sécrétion d'endoglucanase A active dans Bacillus subtilis. La protéine hétérologue sécrétée représente la protéine majoritaire du surnageant de culture de la souche Bacillus subtilis sacU[h].

La séquence NH$_2$-terminale a révélée trois sites de clivage différents dans le voisinage de la séquence de reconnaissance de la signal peptidase. Il apparaît que le nombre de copies du gène n'est pas un facteur limitant pour l'expression de gènes hétérologues.

On a décrit à l'exemple 1 l'expression inductible par le saccharose du gène celA de Clostridium thermocellum dans Bacillus subtilis sur le vecteur multicopie pC 194 en utilisant les signaux de régulation et de sécrétion de la lévane-saccharase de Bacillus subtilis. Cette expression est régulée par au moins trois loci : sacQ, sacS et sacU[21]. Le produit du gène celA, l'endoglucanase A a été sécrété et une haute activité enzymatique a été retrouvée dans le surnageant de culture d'une souche de Bacillus subtilis sacU[h] transformée et induite par le saccharose (exemple 1). Ces constructions ont révélé une instabilité structurale importante qui peut être due à l'intermédiaire simple brin de pC194 généré durant sa réplication dans Bacillus subtilis[40]. Cette instabilité du plasmide complique les développements biotechnologiques et peut être évitée en introduisant les constructions dans le chromosome où il a été montré que la recombinaison est 1000 fois moins fréquente que dans des plasmides[41].

En outre, l'amplification d'un gène hétérologue dans le chromosome vise à augmenter l'expression. On sait qu'une construction dans le chromosome de Bacillus subtilis consistant en un marqueur génétique encadré directement par des séquences dupliquées, peut conduire à une amplification du marqueur et d'une des ces séquences dupliquées (constituant ainsi une unité d'amplification) après application d'une pression de sélection[43–44]. Un maximum de 20 à 50 copies d'unités d'amplification par chromosome peut être obtenu en utilisant une sélection avec un marqueur antibiotique[44]. L'amplification de gène chromosomique dans Bacillus subtilis peut être stimulée par induction de la réplication du plasmide pE194 adjacent qui contient une origine de réplication fonctionnelle dans Bacillus subtilis naturellement thermosensible. Cette amplification intervient sans pression de sélection et donne un nombre de copies d'unités d'amplification jusqu'à 250 copies par chromosome.

Selon l'invention, un fragment de 2,7 kbp d'ADN du plasmide pGJ5 (exemple 1) a été inséré dans l'unité d'amplification introduite dans le chromosome de Bacillus subtilis sacU[h] (souche déposée à la CNCM n° I-744). Ce fragment d'ADN contient le promoteur du gène de la lévane-saccharase Bacillus subtilis (sacB) et la région codant sa séquence signal; fusionné en phase à la séquence codant la séquence mature de l'EGA de C. thermocellum. On se réfère à ce fragment d'ADN ci-après comme étant le gène de fusion celA. On a observé qu'une hypersécrétion de l'EGA était obtenue avec de hauts niveaux d'amplification génique. L'EGA constitue dans ce cas la protéine majoritaire sécrétée par Bacillus subtilis sacU[h]. L'analyse de la partie NH$_2$-terminale de l'EGA sécrétée a révélé trois sites de clivage différents au voisinage de la séquence de reconnaissance de la signal-peptidase. Les cinétiques d'amplification ont été suivies en déterminant le nombre de copies de l'unite d'amplification. L'activité enzymatique des protéines codées par la structure amplifiée a révélé un délai entre le début de l'amplification des gènes et leur expression.

## 2. Amplification du gène celA

Deux stratégies d'amplification de l'ADN chromosomique ont été développées pour augmenter le nombre de copies du gène dans Bacillus subtilis comme alternative à des vecteurs multicopies qui sont souvent instables.

### 2.1 Amplification du gène dans la souche AP551i

Cette souche contient le gène de résistance au chloramphénicol (Cm[r]) et le gène de fusion celA encadrés par des copies de pBR322 (voir ci-après 6. le protocole expérimental) (figure 9). Dans cette souche, l'origine de réplication de pE194 est inactive, comme l'indique le "i" suivant le label de la souche. La structure est intégrée dans la région thyB du chromosome de Bacillus subtilis sacU[h45]. Des duplications de ce type produisent des structures amplifiées stables mais à faible fréquence. Les clones amplifiés peuvent être sélectionnés[44]. Des cellules de AP551i ont été étalées sur du chloramphénicol (50 μg/ml) pour sélectionner les cellules amplifiées et les cellules résistantes ont été isolées (1 pour 10[5]). L'ADN chromosomique de 4 clones sélectionnés a été analysé et on a constaté qu'il contenait 20 à 30 copies de l'unité d'amplification tel que visualisé par électrophorèse sur gel après hydrolyse avec PvuII (figure 10). Cette enzyme de restriction clive une seule fois l'unité d'amplification conduisant à une bande fortement fluorescente de 8,9 kbp qui se distingue des autres bandes faiblement fluorescentes dans chaque ADN amplifié et qui est absente dans la souche parentale. L'expression du gène celA amplifié a été dosée après induction par le saccharose. L'intensité de la bande d'amplification (voir protocole expérimentale) (figure 10) des clones testés a été corrélée avec les tailles de halo résultant de l'hydrolyse de la CMC (voir protocole expérimental) autour des colonies visualisées par le test au rouge Congo sur boîte de Petri. La stabilité des clones a été analysée dans le milieu liquide en présence de saccharose avec et sans pression de sélection (Cm). Les activités CMC-ase des surnageants ont été dosées et étaient indistinctes pour chaque isolat en présence ou en l'absence de Cm, confirmant la stabilité de l'amplification[44]. L'activité EGA dans le surnageant des clones présentant le plus grand nombre de copies de l'unité d'amplification était de 0,8 U/ml.

### 2.2 Amplification de gène dans la souche AP551

Cette souche est isogénique avec la souche AP551i excepté qu'elle contient une copie du plasmide pE194 fonctionnelle dans le chromosome[46]. L'orientation de pE194 dans AP551 est telle que sa réplication qui est unidirectionnelle[47], progresse vers la duplication (figure 9). Quand la réplication de pE194 est active à 37°C, on observe que l'amplification de l'ADN de l'unité d'amplification est induite. Pour augmenter le nombre de copies de gènes celA, l'amplification de la souche AP551 a été effectuée en deux étapes. Dans la première étape, on a isolé des souches AP551 contenant environ 20 copies de l'unité d'amplification obtenue par sélection comme décrit ci-dessus pour AP551i. Cette étape a été effectuée à 51°C, température à laquelle le réplicon pE194 est inactif. Dans la seconde étape, ces isolats ont été incubés dans le milieu liquide à 37°C sans antibiotique en présence de saccharose. A cette température, le réplicon pE194 est actif et une amplification de l'ADN est induite. Une moyenne de 250 copies de l'unité d'amplification par chromosome est observée après 25 heures. Ceci est visualisé par électrophorèse sur gel de l'ADN chromosomique hydrolyse avec PvuII, révélant une bande fluorescente particulièrement forte de la taille attendue de l'unité d'amplification (figure 10). L'activité EGA des surnageants a été dosée et était d'environ 3.6 U/ml pour tous les clones testés.

### 3. Analyse de l'EGA sécrétée

L'analyse des surnageants de culture de souches de Bacillus subtilis AP551 induite par le saccharose, par électrophorèse sur gel de SDS-polyacrylamide (PAGE) a révélé une bande de protéine majoritaire avec une masse moléculaire estimée à 46 000 (figure 11). La masse moléculaire de cette bande est compatible avec celle calculée de la séquence mature de EGA qui est de 49 125 telle que déduite à partir de la séquence nucléotidique[5]. Cette bande est apparue comme étant celle de l'EGA par séquençage de l'extrémité $NH_2$-terminale après SDS-PAGE et transfert comme décrit par Matsudaira[48]. Le transfert du gel de polyacrylamide sur un gel d'agarose contenant de la CMC a montré que la protéine est dotée d'activité CMC-ase (figure 11).

Les données concernant la séquence $NH_2$-terminale (figure 12) indiquent qu'il y a plusieurs sites de clivage aux extrémités $NH_2$-terminale de la protéine mature et seulement 30 % du produit est clivé au site exact prévu. Trois sites de clivage du peptide signal majoritaires ont été détectés, et dans tous les cas le site de clivage était à l'extrémité COOH-terminal d'un résidu alanine et dans le voisinage du site de reconnaissance de la signal peptidase. Les deux derniers résidus phénylalanine-asparagine de la séquence signal de lévane-saccharase sont présents pour 45 % du produit. Dans 25 % du produit, un résidu était manquant en comparaison à la séquence de l'EGA mature (figure 12).

L'activité CMC-ase des surnageants de culture a été correlée avec l'activité spécifique de EGA et l'analyse des surnageants par SDS-PAGE semble indiquer que les trois formes de la protéine sont actives. Même dans un mutant sacU[h] qui est connu pour surproduire des protéases[45] il n'y a pas eu de perte d'activité CMC-ase observée pendant une incubation de 25 heures des cellules en croissance jusqu'en phase stationnaire. Ceci indique que l'EGA était résistante à la dégradation protéolytique. L'EGA est donc sécrétée dans le milieu de culture sous une forme active et stable.

Dans la souche AP551, le gène de structure de la lévane-saccharase sacB est présent. Le surnageant de culture de AP551 induit par saccharose a été dosé par son activité de lévane-saccharase endogène avant et après amplification. Les isolats amplifiés produisent au moins 20 fois moins d'activité lévane-saccharase que les isolats non amplifiés. Ceci confirme l'analyse SDS-PAGE qui montre que la lévane-saccharase est la protéine sécrétée majoritaire dans les souches contrôles (résultat non présenté) mais n'est pas détectée dans la souche AP551 amplifiée (figure 11). Ceci traduit un effet de compétition entre le gène sacB et le gène de fusion celA recombinant pour son expression ou sa sécrétion (ou les deux).

### 4. Relation cinétique entre l'amplification du gène et l'expression

L'expression de deux gènes, le gène de fusion celA et le gène Cm[r] tous deux contenus dans l'unité d'amplification, a été analysée. Le gène Cm[r] code pour la chloramphénicol acétyltransférase (CAT) de pC194 qui est intracellulaire[49]. On a fait croître des cellules AP551 dans un milieu nutritif supplémenté en saccharose sans antibiotique pendant 25 heures à 37°C, température à laquelle l'amplification est induite. Les aliquotes ont été retirés après 0, 2, 3, 4, 5, 17 et 25 heures de croissance. Le contenu en ADN, l'activité CAT dans les extraits cellulaires et l'activité EGA dans les surnageants ont été analysés. Les résultats sont présentés figure 13.

Le nombre de copies de l'unité d'amplification a augmenté rapidement de 25 à 200 copies par chromosome pendant les cinq premières heures de croissance à 37°C. Ensuite, le taux d'amplification a ralenti et une moyenne de 250 copies par chromosome a été atteinte après 25 heures. L'activité CAT est restée constante pendant les premières heures de croissance et a ensuite augmenté correspondant à une augmentation totale de l'activité CAT d'un facteur 50. Un décalage de quelques heures se produit aussi avant l'augmentation de l'activité EGA. L'augmentation totale en activité EGA a été de 10 fois.

## 5. Discussion

Nous avons étudié l'expression du gène de fusion celA après amplification dans le chromosome de Bacillus subtilis. Le gène de fusion celA et le gène marqueur Cm[r] ont été introduits dans la région thyB du chromosome de Bacillus subtilis sacU[h] encadré par une séquence dupliquée de 3,9 kbp. L'amplification a été réalisée dans deux souches isogéniques AP551 et AP551i différant entre elles par la présence ou l'absence respectivement d'une origine de réplication conditionnelle, dans le voisinage de la duplication. Dans les deux cas, une amplification d'un facteur 20 des gènes a été obtenue par sélection des clones résistants à 50 µg/ml de Cm sur des boîtes de Petri. Dans la souche AP551, l'activation de l'origine de réplication pE194 a permis une amplification additionnelle passant de 20 à 250 copies des gènes par chromosome sans pression de sélection. La structure amplifiée représente environ 50 % du chromosome (l'unité d'amplification et le chromosome ayant respectivement 9 kbp et 5000 kbp). De tels niveaux d'ADN amplifiés par cellules n'ont jamais été obtenus dans Bacillus subtilis auparavant et peuvent être comparés au niveau de l'amplification observée dans l'espèce Streptomyces[50]

Les plus hauts niveaux d'amplification de gène conduisent aux plus hauts niveaux d'expression de EGA sécrétée. Dans le surnageant des isolats amplifiés, l'activité CMC-ase de l'enzyme peut être correlée à son activité spécifique[24] (140 U/mg) et correspondait à une concentration de protéine d'environ 10 mg/l/unité DO660, ce qui est 10 fois plus que les taux obtenus précédemment avec la même construction génique sur un vecteur plasmidique multicopies (voir exemple 1). L'analyse de la séquence $NH_2$-terminale de EGA a révélé trois sites de clivage, et le clivage exact a lieu dans 30 % des cas. Toutes les autres formes correspondent à des hydrolyses au niveau d'alanine présentes au voisinage de la zone de clivage (figure 12).

Les conséquences de l'amplification de l'ADN induites par la réplication de pE194 sur l'expression du gène de fusion celA et du gène marqueur Cm[r] ont eté analysées. L'activité des deux enzymes augmente lentement pendant les premiers stades d'amplification, ce qui correspond à la croissance logarithmique. Toutefois, avec l'entrée en phase stationnaire et le ralentissement de l'amplification, les niveaux d'expression des deux gènes ont augmenté fortement. Il apparaît donc y avoir un temps de retard entre l'augmentation du nombre de copies de gènes et l'augmentation de l'expression. Il est intéressant de noter que l'amplification d'ADN intervient activement pendant la phase de croissance exponentielle tandis que l'expression efficace des gènes amplifiés intervient durant la phase stationnaire. Bien qu'il soit difficile de comparer le niveau d'expression des deux gènes exprimés à partir de différents promoteurs et soumis à différents mécanismes de régulation, les nombres de copies de gènes et les niveaux d'expression ne semblent pas être proportionnels. En outre, l'augmentation de l'activité exprimée à partir des deux gènes n'est pas semblable. L'activité CAT augmente d'un facteur 50 en 25 heures, tandis que l'activité EGA augmente seulement d'un facteur 10, suggérant que d'autres facteurs limitants peuvent moduler l'efficacité du procédé.

En conclusion, il apparaît qu'une amplification d'ADN selon l'invention est une bonne alternative au vecteur d'expression multicopie classique, spécialement dans Bacillus subtilis où ceux-ci sont souvent instables. Les résultats présentés ci-dessus indiquent qu'il est possible d'augmenter le nombre de copies de gènes hétérologues dans Bacillus subtilis à de très hauts niveaux et que la machinerie cellulaire est capable de transcrire et traduire cet ADN additionnel. Ce système permet donc l'amplification de gènes hétérologues à des niveaux où le nombre de copies de gènes n'est pas un facteur limitant pour l'expression de ces gènes.

## 6. Le protocole expérimental

### Plasmides et souches bactériennes

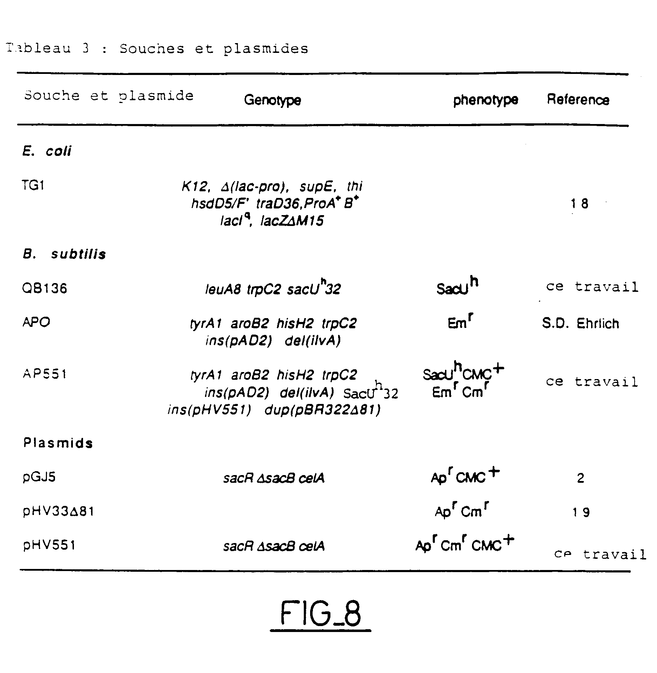

Ces plasmides et souches sont énumérés dans le tableau 3 (figure 8). Leurs constructions sont décrites ci-après.

### 6.1 Transformation et milieux

Pour la croissance d'Escherichia coli un milieu nutritif Luria[22] supplémenté avec de l'ampicilline (Ap) (100 µg/ml) ou du chloramphénicol (Cm) (10 µg/ml) a été utilisé. Pour la croissance de Bacillus subtilis, un milieu nutritif de sporulation[30] a été utilisé, supplémenté avec du Cm (5-50 µg/ml) ou de l'érythromycine (0,3 µg/ml) comme approprié. Le saccharose a été ajouté aux milieux pour l'induction du promoteur sacB dans Bacillus subtilis à une concentration finale de 2 % (w/v). La transformation de E. coli a été effectuée selon Cohen et al[9] et la transformation de Bacillus subtilis a été effectuée tel que décrit par Anagnostopoulos et Spizisen[2].

### 6.2 Dosages d'enzyme

La carboxyméthylcellulose (CMC) (N° C-4888 viscosité moyenne, sigma Co) a été utilisée comme substrat pour le dosage de l'activité EGA. La détection d'activité endoglucanase par le test au rouge Congo sur boîtes de Petri a été effectuée tel que décrit par Cornet et al[10]. Le dosage quantitatif de EGA a été effectué dans un tampon PC (50 mM $K_2HPO_4$, 12 mM acide citrique, pH 6,3) à 60°C. L'activité carboxyméthylcellulase (CMC-ase) a été mesurée selon Béguin et al[6]. Une unité d'activité est définie comme la quantité d'enzyme qui libère 1 µmole d'équivalent de glucose par minute. L'activité spécifique CMC-ase a été exprimée en unité par mg de protéine cellulaire totale. La détection de l'activité cellulase dans des gels de polyacrylamide par réplique et coloration au rouge Congo a été effectuée tel que décrit par Béguin[52].

L'activité chloramphénicol acétyltransférase (CAT) a été mesurée par dosage spectrophotométrique tel que décrit par Shaw[49]. L'activité spécifique CAT a été exprimée en nmoles par minute par mg de protéine.

### 6.3 Constructions des souches

La souche AP551 a été construite en deux étapes, selon le schéma figure 9.

### 6.3.1 Construction de la souche APO (figure 9)

Le plasmide pHV1220[47] est composé de
- pE194cop6, plasmide de Bacillus subtilis conférant la résistance à l'érythromycine (Em[r]). Ce plasmide est thermosensible, sa réplication est inactivée à 51°C. Il est représenté par un rectangle noir sur la figure, la flèche indiquant le sens de la réplication,
- pBR322, dont aucune séquence n'est connue pour s'exprimer chez Bacillus subtilis. Il est représenté par une large flèche blanche sur la figure.
- thy et X, deux fragments proches, mais non adjacents du chromosome de Bacillus subtilis, clonés dans la même orientation, l'un par rapport à l'autre, que sur le chromosome. Thy porte le gène thyB situé à 195° sur la carte génétique. Thy et X sont représentés par une ligne noire sur la figure.

Après transformation des cellules compétentes de SB202 par pHV1220, les transformants ont été sélectionnés pour la résistance à l'érythromycine, à 51°C. A cette température en effet, pHV1220 n'étant pas réplicatif, il se comporte comme un vecteur intégratif dans Bacillus subtilis : ce vecteur est capable de recombiner avec le chromosome bactérien dans les régions homologues, thy et X. Lorsque le vecteur s'intègre par double crossing over, entre les séquences "thy" et "X" respectivement, il y a délétion de la partie du chromosome comprise entre les deux loci, et en particulier du gène ilvA. A cette étape de la construction, seuls les transformants issus d'une intégration par double crossing over (détectés par leur phénotype Ilv⁻, 50 % du total) ont été retenus. La souche ainsi contruite a été nommée AP0.

### 6.3.2 Construction de la souche AP551 (figure 9)

pHV551 est un plasmide hybride composé de :
- pBR322,
- le gène de résistance au chloramphénicol de pC194 (Cm[r]),
- le gène de dégradation de la cellulose celA cloné sous contrôle du promoteur sacB, inductible par le saccharose. Sa construction a été décrite dans l'exemple 1.

Au cours de la transformation de AP0 par pHV551, l'ADN chromosomique d'une souche sacU[h] a été ajouté. Les transformants ont été sélectionnés pour la résistance au chloramphénicol, à 51°C. Parmi les transformants, ceux ayant acquis par congression la mutation sacU[h] ont été retenus. Cette mutation permet un niveau d'activation du promoteur sacB supérieur a l'allèle sauvage.

Le plasmide pHV551 a été obtenu par ligature de pHV33_81[53] avec le fragment EcoRI-HindIII de 2,7 kbp de pGJ5 (exemple 1). Le plasmide pHv33Δ81 est constitué par une partie de pBR322 et le gène de résistance au chloramphénicol de pC194[16]. Le pHV33Δ81 se réplique dans Escherichia coli mais pas dans Bacillus subtilis. Le fragment EcoRI-HindIII de pGJ5 contient le promoteur et la région codant la séquence signal de la lévane-saccharase de Bacillus subtilis, fusionnée en phase à la séquence codant la séquence mature de l'EGA de C. thermocellum. Le plasmide résultant appelé pHV551 a été intégré dans le chromosome de Bacillus subtilis souche APO et a généré la souche AP551 (figure 9) par transformation et sélection des transformants Cm[r].

pHV551 n'est pas réplicatif dans Bacillus subtilis. Il se comporte donc en vecteur intégratif, capable de recombiner avec le chromosome bactérien de AP0 dans la région d'homologie, pBR322. Lorsque le plasmide s'intègre, par simple crossing over, il y a duplication de sa région d'homologie, pBR322.

La souche résultante AP551 comporte donc, dans la région thyB de son chromosome :
- le plasmide pE194 cop6, dont la réplication est inactivée à 51°C, et fonctionnelle à 37°C,
- les gènes celA et Cm[r] encadrés par une duplication de 3,9 kb des séquences de pBR322. Cette duplication délimite l'unité d'amplification (A.U.), composée d'une des séquences répétées et des deux marqueurs celA et Cm[r]. C'est cette unité d'amplification qui est amplifiée jusqu'à 250 fois dans les expériences décrites précédemment.

Plus précisément, la souche AP551 porte dans la région thyB de son chromosome une structure comprenant : (a) le plasmide pE194 cop-6 (ci-après pE194) qui est naturellement thermosensible pour la réplication[46]; (b) une séquence dupliquée de 3,9 kbp de pBR322 (pBR322 ne se réplique pas dans Bacillus subtilis[54]; (c) un segment de 2,3 kbp contenant le gène de résistance au Cm et (d) un segment de 2,7 kbp contenant le gène de fusion celA. Les deux séquences de (b) sont situées de part et d'autre de (c) et (d) et définissent l'unité d'amplification, un élément de 8,9 kbp composé d'un segment de pBR322 et des gènes Cm[r] et gène de fusion celA. L'orientation de pE194 dans les cellules AP551 est telle que la réplication initiée à son origine progresse en direction de la réplication[47].

La souche AP551i a été construite selon la même procédure : cette souche est isogénique avec la souche AP551 excepté le fait que l'origine de réplication pE194 est inactivée suite à une délétion de 200 bp dans sa région[47]. En outre, la mutation sacU[h] a été introduite dans les souches AP551 et AP551i par congression avec l'ADN chromosomique de la souche sacU[h] QB 136. Les mutants sacU[h] ont été isolés par criblage sur des milieux révélant une surproduction de protéase et lévane, caractéristique des mutants sacU[h][45]. La construction correcte des souches AP551 et AP551i a été vérifiée par analyse selon Southern de l'ADN chromosomique en utilisant pHV33Δ81 comme sonde. Des études ont été effectuées avec des clones portant avant amplification seulement une copie de pHV551.

### 6.4 Analyse d'ADN

L'ADN chromosomique a été extrait des lysats cellulaires avec du phénol-chloroforme suivi d'une précipitation avec de l'isopropanol et un traitement à la RNase des acides nucléiques.

La détection d'ADN amplifié a été effectuée par analyse de restriction de l'ADN chromosomique. L'hydrolyse avec PvuII fournit un fragment de 8,9 kbp correspondant à l'unité d'amplification qui, quand le niveau d'amplication est élevé, peut être distinguée sous forme d'une bande séparée sur gel d'agarose (figure 10).

Le niveau d'amplification de l'ADN a été mesuré par "dot blots". Pour de hauts niveaux d'amplification (plus de 20 copies par chromosome), des échantillons d'ADN ont été dilués 10 à 100 fois dans de l'ADN chromosomique du type sauvage avant l'analyse. pC194 qui est homologue en partie de l'unité d'amplification, a été utilisé comme sonde et le gène sacA[55] a été utilisé comme sonde chromosomique de référence, correspondant à une copie par chromosome du gène de la saccharase de Bacillus subtilis.

### 6.5 Abréviation

Le gène celA est le gène de structure pour l'endoglucanase préalablement purifiée à partir de C. thermocellum NCIB10682[23] qui est désignée endoglucanase A. La carboxyméthycellulose (CMC) est un substrat spécifique des endoglucanases. L'activité endoglucanase A est assimilée à l'activité carboxyméthylcellulase (CMC-ase).

**EXEMPLE 3: Hyperproduction en fermenteur pilote (20 litres) de cellulase de clostridium thermocellum et de chloramphénicol-acétyl transférase de staphylococcus aureus à partir de bacillus subtilis par induction de l'amplification de l'ADN chromosomique.**

## 1. Introduction

De façon à tester la faisabilité industrielle des productions d'EGA et de CAT par le système d'amplification chromosomique développé dans B.subtilis, des cultures de la souche AP551 induite par le saccharose ont été réalisées en fermenteur de 20 litres.

La souche utilisée pour ces essais a préalablement subi la première étape de l'amplification chromosomique, c'est-à-dire une sélection à 51°C sur milieu gélosé contenant du chloramphénicol 50 µg/ml (cf. exemple 2).

Les colonies qui se développent sur ce milieu sélectif sont reprises pour ensemencer une préculture qui constituera l'inoculum du fermenteur. La préculture est effectuée en milieu liquide sans antibiotique ni saccharose, elle est incubée à 51°C.

Ainsi, le fermenteur de 20 litres est ensemencé avec la souche AP551 dont l'ADN chromosomique contient environ 20 copies de l'unite d'amplification.

Le milieu de culture du fermenteur contient du saccharose, c'est donc au stade du fermenteur que l'expression des gènes cel A et cm$^r$ est induite (cf. exemple 1).

L'amorce de la croissance en fermenteur est effectuée à 51°C. Puis la température est abaissée à 37°C. Ceci constitue la deuxième étape de l'amplification qui permet a l'unité d'amplification d'atteindre plus de 200 copies par cellule (cf. exemple 2).

## 2. Matériel et méthodes

### 2.1 Microorganisme

Bacillus subtilis Souche AP551.

### 2.2. Milieux de culture

Milieu gélosé sélectif
bactotryptone 12,5 g/l
Extrait de levure 24 g/l
Glycérol 4 ml/l
KH2PO4 2,3 g/l
K2H PO4 12,54 g/l
chloramphénicol 50 µg/ml

Milieu de préculture

identique au milieu ci-dessus mais sans antibiotique

Milieu de production

Extrait de levure 10 g/l
biotrypcase 12 g/l
KCL 20 mg/l
KH2PO4 200 mg/l
K2H PO4 100 mg/l
MnSO4 10 mg/l
CaCl2 9 mg/l
MgSO4 7H20 17 mg/l
Fe SO4 1 mg/l
(NH4)2 SO4 4 g/l
Saccharose 40 g/l

2.3 Technique de culture

Fiole conique : les précultures sont effectuées en fioles coniques de 500 ml contenant 150 ml de milieu. Les fioles sont agitées et incubées à 51°C.

Fermenteurs : la production d'enzyme est effectuée en fermenteur de 20 litres contenant 15 litres de milieu de production.

Le taux d'ensemencement est de 2% (v/v); le pH est régulé à 7 par NH4OH 20%; l'oxygène dissous est régulé à 20% par l'agitation qui varie de 400 à 900 rpm, le débit de l'aération est fixé à 1vvm; la température est de 51°C en début de croissance puis lorsque la densité optique (à 660 nm) atteint une valeur de 0,2 la température est abaissée à 37°C.

Une alimentation discontinue de saccharose est pratiquée sous la forme de deux ajouts de 0,7 litres de saccharosetitrant 300 g/l effectués entre les temps 15 et 20 heures pour le premier et 25 et 30 heures pour le second.

2.4 Dosages d'enzyme et analyse d'ADN

Cf. exemple 2.

**3. Résultats**

La culture en fermenteur de 20 litres a été réalisée sur une période de 40 heures.

La croissance cellulaire, l'activité EGA des surnageants, l'activité CAT des extraits cellulaires, l'évolution du nombre de copies de l'unité d'amplification ont été mesurées à partir de prélèvements de moût de culture effectués aux temps 0 - 1,5 - 16 - 20 - 25 et 40 heures (figure 14). Le changement de température de 51 °C à 37°C a été opéré au temps 1,5 heures.

3.1 Croissance cellulaire

Le suivi de la croissance cellulaire montre que la biomasse est maximale, avec une densité optique de 15 (mesurée à 660 nanomètres) à 25 heures. Puis la biomassee reste sensiblement constante jusqu'à 40 heures.

3.2 Activité EGA

L'activité CMCase des surnageants croît de 0.05 U/ml à 27U/ml entre 0 et 25 heures. En fin de culture, à 40 heures, l'activité CMCase est de 30 U/ml.

3.3 Activité CAT

L'activité spécifique CAT intracellulaire croît rapidement de 15 à 1010 U/mg entre 0 et 25 heures, à 40 heures cette activité atteint 1050 U/mg.

3.4. Amplification chromosomique

Le nombre de copies de l'unité d'amplification augmente de 16 à 250 copies par chromosome pendant les quatorze premières heures de croissance à 37°C, puis il diminue progressivement.

**4. Discussion**

Nous avons réalisé les productions d'EGA, enzyme extracellulaire, et de CAT, enzyme intracellulaire en fermenteur pilote de 20 litres. Nous avons utilisé la souche AP551 de Bacillus subtilis possédant les gènes cel A et cm$^r$ amplifiés dans le chromosome.

La chaîne d'ensemencement a été effectuée avec la souche dans laquelle l'unité d'amplification est portée à 20 copies. L'amplification additionnelle des gènes cel A et cm$^r$ qui augmente à 250 leur nombre de copies par chromosome, a été réalisée dans le fermenteur.

Les résultats obtenus sont résumés dans le tableau suivant. A titre de comparaison, nous avons rappelé les résultats mentionnés dans l'exemple 2 et obtenus avec la souche AP551 cultivée en fiole dans 100 millilitres de milieu nutritif.

|  | Fermenteur | Fiole |
|---|---|---|
| Biomasse en fin de culture D.O 660 nm | 15 | 3 |
| EGA Activité dans le surnageant U/ml de surnageant | 30 | 3,6 |
| EGA Activité spécifique U/mg de protéine cellulaire | 8 | 4,8 |
| CAT Activité dans le moût U/ml de surnageant | 3940 | 675 |
| CAT Activité spécifique U/mg de protéine cellulaire | 1050 | 900 |
| Nombre maximum de copies de l'unité d'amplification | 250 | 250 |

L'amplification chromosomique des gènes EGA et CAT est identique lorsque la croissance est réalisée en fiole ou en fermenteur. Cependant en fermenteur, dans des conditions de croissance prolongée, le nombre de copies tend à diminuer.

La production d'EGA secrétée dans le surnageant est augmentée d'un facteur 8 par rapport à la culture en fiole. Cette augmentation résulte d'une meilleure activité spécifique (x 1.6) mais surtout de la formation d'une biomasse plus abondante (x 5).

En considérant l'activité spécifique de la CMCase purifiée (140 U/mg) la production d'EGA en fermenteur correspondrait à une concentration de protéine d'environ 14 mg/l/unité de D.O., soit 214 mg/l de surnageant.

La production de CAT par unité de volume de moût de fermentation est augmentée d'un facteur 5. Ceci ne résulte que de l'augmentation de la quantité de biomasse produite, les activités spécifiques CAT obtenues en fiole et en fermenteur étant équivalentes.

En conclusion; les résultats présentés ci-dessus montrent que le système d'amplification chromosomique de gènes hétérologues développé dans une espèce de Bacillus et décrit plus haut, conserve son efficacité dans des conditions préindustrielles de culture.

De plus, les productions d'EGA et de CAT ont été fortement augmentées. Ces améliorations portant à la fois sur une enzyme sécrétée et une enzyme intracellulaire sont liées pour l'essentiel à l'obtention de quantité plus importante de biomasse. L'utilisation de fermenteur permet de mieux contrôler et de maximiser la croissance cellulaire.

## EXEMPLE 4: Contrôle de la fonction de recombinaison homologue

Les événements de duplication et d'amplification de gènes chez les procaryotes se produisent au niveau de segments d'ADN encadrés par des régions répétées (56). Il a été démontré (43) que chez B. subtilis la formation d'un domaine amplifié stablement intégré dans le chromosome nécessite la fonction de recombinaison gouvernée par le gène recE. Cependant, l'approche expérimentale de l'amplification de gènes décrite jusqu'à présent étudiait un phénomène ne se produisant que dans de rares cellules et impliquait la sélection de clones se développant à une fréquence faible (de l'ordre de 10-5) et portant un domaine amplifié correspondant à la sélection choisie.

L'approche de l'amplification de gènes selon l'invention diffère en ce que le domaine amplifiable est placé à proximité de l'origine de réplication notamment du plasmide pE194 intégré dans le chromosome dont l'activation (à37°C) de la réplication (thermosensible) induit une amplification massive qui se développe rapidement (cf. exemple 2).

Il n'était donc pas évident que le déclenchement de l'amplification massive induite par l'activité réplicative notamment du plasmide pE194 intégré soit sous la dépendance du gène recE. Selon l'invention, il est montré que l'amplification étudiée ne se développe pas dans un contexte génétique où le produit du gène recE n'est pas exprimé et que la capacité d'amplification est restaurée si la fonction de recombinaison est complémentée. Le contrôle de l'expression de la fonction de recombinaison peut donc être utilisé comme un niveau de contrôle supplémentaire du développement de l'amplification.

Construction des souches

La souche AP2 (cf. tableau 1), qui a été utilisée dans cet exemple, porte un domaine amplifiable qui ne diffère de celui de la souche AP551 (cf. figure 9, exemple 2) qu'en ce que le segment encadré par les copies pBR322 est plus court (segment de 2,3 kb) et ne code que pour la chloramphénicol acétyl transférase (CAT). L'amplification dans la souche AP2 se développe de façon analogue à ce qui a été décrit pour la souche AP551 dans l'exemple 2.

Construction des souches APrec par congression

Le gène recE de la souche AP2 a été inactivé selon deux méthodes :
1 - Congression de l'ADN chromosomique d'une souche AP2 et d'une souche de B. subtilis 168 portant la mutation recE4 (MI112) dans une souche réceptrice (SB202), puis caractérisation des transformants portant à la fois la construction amplifiable de la souche AP2 et la mutation recE4.
2 - Transformation de la souche AP2 avec l'ADN de la souche SBEK et sélection des transformants ayant perdu le gène recE natif par substitution de gène. Cette dernière méthode a été rendue possible par la construction de la souche SBEK décrite dans cet exemple.

Construction des souches APrec par congression

Les souches utilisées sont décrites dans le tableau 1. Les transformants obtenus par congression de l'ADN chromosomique des souches AP2 et MI112 dans la souche réceptrice SB202 ont d'abord été sélectionnés sur chloramphénicol (bug/ml) puis testés, par repiquages, pour leur sensibilité à la mitomycine C (40 ng/ml). Toutes les étapes de sélection ont été conduites à 51°C.

L'acquisition de la mutation recE4 ne pouvant être sélectionnée directement, l'ADN de la souche MI112 a été utilisé en congression à des doses 5 et 10 fois plus importantes que la quantité d'ADN de la souche AP2. 21 clones sensibles à la mitomycine C sur 1500 clones testés par repiquage ont été caractérisés. Après vérifications par striage, seuls 7 clones ont été conservés et deux de ces clones, nommés APrec4 et APrec 18, ont été étudiés plus en détail.

Construction de la souche SBEK

Un fragment chromosomique portant le gène recE de B.subtilis a été cloné dans un phage Φ 105 et il a été montré que le phage recombinant complémente la mutation recE4 (57). A partir d'éléments de séquences dont nous disposions, des oligonucléotides amorce ont été synthétisés et un fragment de 1,1kb du gène recE a pu être amplifié par la méthode dite PCR (58) en utilisant l'ADN du phage recombinant 105rec1 (57) come substrat. L'un des oligonucléotides amorce utilisés contenait la séquence du du site de restriction Kpn1 et l'autre oligonucléotide amorce contenait la séquence du site de restriction HindIII, de telle sorte, de telle sorte que le fragment amplifié a pu être cloné entre les sites Kpn1 et HindIII du vecteur de clonage pUC19. Il est à noter que, le fragment amplifié portant un autre site HindIII, c'est après une digestion partielle HindIII quel le fragment entier (1,1 kb) a pu être cloné dans pUC19. Ce fragment du gène recE contient un site Cla1 unique (position 291 par rapport au site Kpn1) et c'est dans ce site qu'a été inséré un segment Cla1-Cla1 de 1,5 kb portant le gène aphA3 du transposon Tn1545 qui confère la résistance à la kanamycine (35).

Le plasmide ainsi construit, portant une insertion dans la séquence du gène recE, a été linéarisé au site unique Sca1 du vecteur pUC19 (il n'y a pas de site Sca1 dans le fragmentrecE). La souche SB202 a été transformée avec cet ADN linéaire, les transformants étant sélectionnés sur kanamycine (10 μg/ml). La linéarisation du plasmide fait que l'intégration du gène aphA3 au locus chromosomique recE s'effectue par un double événement de recombinaison. L'un des transformants dont la structure chromosomique a été vérifiée par "southern blotting" a été nommé SBEK. La souche SBEK porte donc, par construction, un gène recE inactivé par insertion d'un fragment de 1,5 kb, conférant par ailleurs la résistance à la kanamycine (10 μg/ml). Des tests de sensibilité à la mitomycine C ont montré qu'une dose de 20 ng/ml de mitomycine C est léthale pour la souche SBEK.

L'ADN de la souche SBEK peut être utilisé pour inactiver le gène recE de n'importe quelle souche de Bacillus pourvu que la souche puisse être transformée avec l'ADN chromosomique de la souche SBEK et les transformants sélectionnés sur kanamycine (10 μg/ml). En particulier, la souche AP2 a été rendue compétente (2) et transformée avec l'ADN de la souche SBEK. Les transformants obtenus sur kanamycine (10 μg/ml) ses ont tous révélés sensibles à la mitomycine C (20 ng/ml), alors que la souche AP2 est résistante à cette dose de mitomycine C.

## Résultats :

### 1. L'amplification est bloquée dans les souches APrec

La structure chromosomique des 2 clones APrec construits par congression a été analysée par southeern blotting en utilisant le plasmide pHV33Δ81 comme sonde. Le clone APrec4 porte 2 unités d'amplification (une unité d'amplification de plus que la souche AP2) et le clone APrec18 porte 3 unités d'amplification.

L'ADN de la souche AP2 et des 2 souches APrec cultivées pendant 2 hrs à 37°C et à 51°C a été analysé par électrophorèze sur gel d'agarose en champ pulsé après restriction et southern blotting, la sonde utilisée étant le plasmide pHV33 81 qui couvre toute l'unité d'amplification. Le résultat (figure 15) montre clairement qu'aucun des 2 clones APrec ne produit de matériel amplifié à 37°C, contrairement à la souche AP2 qui sert de contrôle. Le résultat s'analyse comme suit :

- la digestion Nco1 produit 2 fragments de jonction qui comigrent à 13,5 kb et un fragment de 6,2 kb qui correspond à l'unité d'amplification; l'intensité de cette bande, par rapport à la bande des fragments de jonction, est nettement plus intense pour le matériel de la souche AP2 a 37°C; par contre, il n'y a pas de différence détectable dans l'intensité relative de ces bandes pour les souches APrec. La bande d'amplification de 6,2 kb est inattendue à 61°C dans le cas de la souche AP2 et indique un début d'amplification, probablement causé par une température d'incubation sensiblement inférieure à 51 °C. Dans le cas des souches APrec la bande de 6,2 kb est due à la présence de plusieurs unités d'amplification comme le confirme l'analyse Bg1 II.
- la digestion Bg1 II, en l'absence d'amplification ne produit qu'un seul fragment portant l'ensemble du domaine amplifiable. C'est ce que l'on observe avec les souches APrec. Le matériel de la souche AP2 à 51 °C montre une bande majoritaire et une échelle de bandes minoritaires qui correspondent à des intermédiaires d'amplification.

Les expériences de complémentation qui suivent démontrent aussi que cette absence d'amplification n'est pas due à une inactivation fortuite de l'activité réplicative de pE194 au cours de la construction des souches APrec.

### 2.L'amplification peut être restaurée dans les souches APrec en complémentant la fonction de recombinaison

#### 2.1 Lysogénisation des souches APrec avec Φ105 rec1

Le phage recombinant Φ 105 rec1 (57) porte un fragment d'ADN chromosomique contenant le gène recE. Des souches APrec lysogènes pour ce phage ont été produites par "spotting", c'est-à-dire qu'une goutte de lysat a été déposée sur un tapis bactérien et qu'un clone se développant au centre de la zone de lyse a été prélevé et purifié. Un clone pour chacune des 2 souches APrec a été complètement caractérisé. Le phénotype des lysogènes est la résistance à une dose de 20 ng/ml de mitomycine C (complémentation de la mutation recE4) et l'immunité à une surinfection par le phage Φ 105. Ces souches contiennent donc une copie du gène recE insérée dans le génome du phage intégré, et une autre copie de ce gène, portant la mutation recE4, au locus recE. Les cinétiques d'amplification conduites avec les lysogènes obtenus pour chacun des 2 clones APrec ont montré que la capacité d'amplification était entièrement restaurée. Ce résultat montre qu'il sera possible d'introduire des constructions du gène recE dans le chromosome afin d'agir sur le taux d'amplification.

#### 2.2 Complémentation par la protéine recA de E.coli

Le plasmide pPL608 (59) se réplique dans B. subtilis (réplicon de pUB110) et porte une copie du gène recA de E. coli sous le contrôle du promoteur spo2 (promoteur fort dans B. subtilis. Un dérivé de ce plasmide pPL608ΔXba, délété du gène de résistance au chloramphénicol, a été construit, ceci afin de pouvoir maintenir la sélection pour le marqueur chloramphénicol porté par la construction chromosomique dans les souches APrec; le petit fragment XbaI de 500 pb a été délété par digestion XbaI de pPL608 et religation sur lui-même du grand fragment après purification sur gel d'agarose. Des bactéries APrec18 compétentes ont été préparées (2) et transformées avec l'ADN du plasmide pPL608 Xba. Les transformants ont été sélectionnés pour la résistance à la kanamycine portée par le plasmide. Les tests d'amplification ont montré une grande quantité de matériel amplifié, particulièrement lorsque la culture à 37°C avait été continuée toute la nuit. L'analyse du domaine amplifiable de la souche maintenue à 51°C a cependant montré que le transformant étudié portait 5 unités d'amplification, une situation qui est propice à une forte amplification (cf. exemple 2). Il n'a donc pas été possible de comparer ces taux d'amplification avec ceux des souches contenant recE mais cette expérience prouve que le gène recA de E. coli complémente le gène recE muté pour le processus d'amplification.

Dans cet exemple, il a été montré que l'amplification dans les cellules AP2 est bloquée lorsque le gène recE est inactivé. Ce résultat n'était pas évident car il était concevable que l'action du produit du gène recE (nécessaire à la production de domaines amplifiés stablement intégrés dans le chromosome) s'exerce à une étape de stabilisation dans le chromosome, postérieure à la production des domaines amplifiés révélés par l'analyse biochimique. L'absence de tout intermédiaire d'amplification dans les analyses sur gel du matériel extrait des souches dans lesquelles le gène recE est inactivé suggère au ocntraire que la fonction de recombinaison participe au déclenchement du processus d'amplification, probablement par un événement de recombinaison au niveau des séquences répétées (pBR 322).

Un autre événement de recombinaison, la délétion du marqueur chloramphénicol par recombinaison entre les séquences pBR322, a aussi été décrit dans les cellules AP2 (47) et les analyses biochimiques par blotting mettent en évidence des fragments chromosomiques correspondant à cette délétion. Aucune bande de délétion n'a été observée sur le matériel des souches APrec, ce qui montre que cet autre événement de recombinaison activé par la réplication de pE194 est aussi bloqué par l'inactivation de recE.

La démonstration qui est faite dans cet exemple que le gène muté recE peut être complémenté par le gène recA de E. coli (placé sous le contrôle d'un promoteur de B. subtilis) ouvre la porte à des recherches de complémentation qui favoriserait l'un des événements de recombinaison par rapport à l'autre (amplification ou délétion). Il est aussi possible que le taux d'expression de la fonction de recombinaison peut donc être considéré comme un second niveau de contrôle qui peut permettre de moduler la réponse recombinogénique à l'activation de la réplication.

## TABLEAU 1 DES SOUCHES ET PLASMIDES

| Souches | Génotype | Phénotype | Réf. |
|---|---|---|---|
| SB202 | trpC2,tyrA1,aroB2,hisH2 | | 30 |
| SBEK | trpC2,tyrA1,aroB2,hisH2 recE: : aphA3 | $Km^R$ $MC^S$ | ce travail |
| \P2 | trpC2,tyrA1,aroB2,hisH2 ins(pAD2) del (IlvA) ins(pHV33$\Delta$81)dupl(pBR322$\Delta$81) | $Em^R$ $Cm^R$ | 47 |
| \I112 | leuA8,arg15,thr5,recE4 r-m- | $Mc^S$ | 60 |
| Plasmides | | | |
| pUC19 | | $Ap^R$ | 61 |
| pPL608 | | $Km^R$ $Cm^R$ | 59 |
| pPL608 Xba | | $Km^R$ | ce travail |

**Le protocole expérimental:**

Amplification d'un fragment d'ADN de 1,1 kb par PCR:

1. Choix des oligonucléotides (20-nucléotides; Appligène)

a - Région promoteur : introduction d'un site KpnI.

recE   G T A G T G G T A A C A T A A A G G A G G A A A A A
                     I

oligo  5'  A G T G G T A C C A T A A A G G A G G A  3'

b - Région stop : introduction d'un site HindIII

```
recE    T T G T A T C A T T T G A A A C T T A T T T A T T
                                              ·

                                              I

oligo   5'  G T A T C A T T T G A A G C T T A T T T T  3'
```

## 2. Réaction PCR :

100 µl de mélange réactionnel, comme indiqué par Pharmacia, contenant :
- 20 ng environ de DNA du phage Φ 105 recl
- oligonucléotides amorce à 1,0 µm
- dNTPs 200 µM chaque
- Tris 10 mM pH 8,3; MgC 12 2mM; 50 mM KCl
- gélatine 0,001%
- 25 unités de Taq polymérase

La réaction est conduite dans l'appareil Pharmacia "Gene ATAQ".

Cycle de température choisi :
- 94°C 1 mn
- 52°C 2 mn
- 72°C 3 mn

La réaction est conduite sur 30 cycles de température :
- départ 94°C 5 mn
- 30 cycles
- Fin 72°C 10 mn

**Conditions d'électrophorèze en champ pulsé :**

Programme "DNA star".

Electrophérèze conduite en tampon Tris-Borate 0,5X, sous 8 V/Cm de tension, pendant 16 hrs. La température est amintenue con stante dans la cuve par un système de refroidissement par circulation d'eau.

Mode d'alternance du champ :
- départ : 0,3/0,1 V
- fin 3/1V

Gel d'agarose 1%

## LITTERATURE CITEE

**1. Amory, A., F. Kunst, E. Aubert, A. Klier, and G. Rapoport.** 1987. Characterization of the sacQ genes from Bacillus licheniformis and Bacillus subtilis. J. Bacteriol. **169**:324-333.

**2. Anagnostopoulos, C., and J. Spizizen.** 1961. Requirements for tranformation in Bacillus subtilis. J. Bacteriol. **81**:741-746.

**3. Aymerich, S., G. Gonzy-Tréboul, and M. Steinmetz.** 1986. 5'-Non-coding region sacR is the target of all identified regulation affecting the levansucrase gene in Bacillus subtilis. J. Bacteriol. **166**:993-998.

**4. Béguin, P.** 1983. Detection of cellulase activity in polyacrylamide gel using Congo red stained agar replicas. Anal. Biochem. **131**:333-336.

**5. Béguin, P.,P. Cornet, and J.-P. Aubert.** 1985. Sequence of a cellulase gene of the thermophilic bacterium Clostridium thermocellum. J. Bacteriol **162**:102-105.

**6. Béguin, P.,P. Cornet, and J. Millet.** 1983. Identification of the endoglucanase encoded by the celB gene of Clostridium thermocellum. Biochimie **65**:495-500.

**7. Béguin,P., M. Rocancourt, M.-C. Chebrou, and J.-P. Aubert.** 1986 Mapping of mRMNA endocing endoglucanase A from Clostridium thermocellum. Mol. Gen. Genet. **202**:251-254.

**8. Carter,P.,H. Bedouelle, and G. Winter.** 1985. Improved oligonucleotide site directed mutagenesis using M13 vectors. Nucleic Acids Res. **13:4431-4443.**

**9. Cohen, S.N., A. C. Y. Chang, and L. Hsu.** 1972. Non chromosomal antibiotic resistance in bacteria genetic transformation of Escherichia coli by R-factor DNA. Proc. Natl. Acad. Sci. USA **69**:2110-2114.

**10. Cornet, P., J. Millet, P. Béguin, and J.-P. Aubert.** 1983. Characterization of two cel (cellulose degra- dation) genes of Clostridium thermocellum coding for endoglucanases. Bio/Technol. **1**:589-594.

**11. Edelman, A., G. Joliff, A. Klier, and G. Rapoport.** 1988. A system for the inducible secretion of proteins from Bacillus subtilis during logarithmic growth. FEMS Microbiol. Lett. **52**:117-120.

**12. Fouet, A., M. Arnaud, A. Klier, and G. RAPOPORT.** 1984. Characterization of the precursor form of the exocellular levansucrase from Bacillus subtilis. Biochem. Biophys. Res. Commun. **119**:795-800.

**13. Gibson, T.J.** 1984. Ph. D. thesis, University of Cambridge.

**14. Honjo. M., A. Akaoka, A. Nakayama, H. Shimada, I. Mita, K. Kawamura, and Y. Furutani.** 1986. Construction of secretion vector and secretion of hIFN-B, p. 89-100. In A. T GANESAN and J. A. Hoch (eds), Bacillus Molecular genetics and Biotechnology Applications. Academic Press, New-York.

**15. Honjo, M., A. Nakayama, H. Shimada, A. Lio, I. Mita, K. Kawamura, and Y. Furutani.** 1988. Construction of an efficient secretion host-vector system in Bacillus subtilis, p. 365-369. In A. T. Ganesan and J. A. Hoch (eds), Genetics and Biotechnology of Bacilli, Academic Press, New-York.

**16. Horinouchi,S., and B. Weisblum.** 1982. Nucleotide sequence and functional map of pC194, a plasmid that specifies inducible chloramphenicol resistance. J. Bacteriol. **150**:815-825.

**17. Klier, A., A. Fouet, M. Débarbouillé, F. Kunst, and G. Rapoport.** 1987. Distinct control sites located upstream from the levansucrase gene of Bacillus subtilis. Molec. Microbiol. **1**:233-241.

**18. Le Coq, D., P. Joyet, B. Le Révérend, and H. Heslot.** 1986. Expression of levansucrase/alpha-amylase gene fusions in Escherichia coli and Bacillus subtilis part B, p. 457-463. In M. Alacevic, D. Hranueli and Z. Toman (eds), Zagreb, Yugoslavia : Pliva, 5Th Int. Symp. Genet. Ind. Microorg., Split, Yugoslavia.

**19. Lepesant, J.-A.** 1974. Thesis, Paris VII.

**20. Lepesant, J.-A., F. Kunst, J. Lepesant-Kejzlarova, and R. Dedonder.** 1972. Chromosomal location of mutations affecting sucrose metabolism in Bacillus subtilis Marburg. Molec. Gen. Genet. **118**:135-160.

**21. lepesant, J.-A., F. Kunst, M. Pascal, J. Lepesant-Kejztarova, M. Steinmetz, and R. Dedonder.** 1976. Specific and pleitotropic regulatory mechanisms in the sucrose system of Bacillus subtilis 168, p. 58-69. In D. Schlessinger (ed.), Microbiology-1976. American Society for Microbiology, Washington, D.C.

**22. Maniatis, T., E.F. Fritsch, and J. Sambrook.** 1982. Molecular cloning, a laboratory manual. Cold Spring Harbor, New-York.

**23. Petre, J., R. Longin, and J. Millet.** 1981. Purification and properties of an endo-bêta-1,4-glucanase from Clostridium thermocellum. Biochimie **63**:629-639.

**24. Pétré, D., Millet, J., Longin, R., Béguin, P., Girard, H., and J.-P. Aubert.** 1986. Purification and properties of the endoglucanase C of Clostridium thermocellum produced in Escherichia coli Biochimi **68**:687-695.

**25. Robson, L.M., and G.H. Chambliss.** 1984. Characterization of the cellulolytic activity of a Bacillus isolate. Appl. Environ. Microbiol. **47**:1039-1046.

**26. Sacco, M., J. Millet, and J.-P. Aubert.** 1984. Cloning and expression in Saccharomyces cerevisiae of a cellulase gene from Clostridium thermocellum Ann. Microbiol. Inst. Pasteur. **135A**:485-488.

**27. Sanger, F., S. Nicklen, and A. R. Coulson.** 1977. DNA sequencing with chain terminating inhibitors. Proc. Natl. Acad. Sci. USA. **74**:5463-5467.

**28. Sarvas, M.** 1986. Protein secretion in Bacilli. Current Topics in Microbiol. and Immunol. **125**:103-125.

**29. Saunders,C. W., B.J. Schmidt, R.L. Mallonee, and M.S. Guyer.** 1987. Secretion of human serum albumin from Bacillus subtilis. J. Bacteriol. **169**:2917-2925.

**30. Schaeffer, P., H. Ionesco, A. Ryter, et G. Balassa.** 1965. La sporulation de Bacillus subtilis, étude génétique et physiologique, P. 553-563. In Mécanismes de régulation des activités cellulaires chez les microorganismes, Paris : Centre National de la Recherche Scientifique, 1965.

**31. Schein, C. H., K. Kashiwagi, A. Fujisawa, and C. Weissmann.** 1986. Secretion of mature IFN-alpha2 and and accumulation of uncleaved precursor by Bacilius subtilis transformed with a hybrid alpha-amylase signal sequence-IFN-alpha2-gene. Bio/Technology **4**:719-725

**32. Schwarz, W. H., F. Grabnitz, and W.L. Staudenbauer.** 1986. Properties of Clostridium thermocellum endoglucanase produced in Escherichia coli Appl. Environ Microbiol. **51**:1293-1299.

**33. Soutschek-Bauer, E., and W.L. Staudenbauer.** 1987. Synthesis and secretion of a heat-stable carboxymethylcellulase from Clostridium thermocellum in Bacillus subtilis and Bacillus stearothermophilus. Mol. Gen. Genet. **208**:537-541.

**34. Steinmetz, M., D. Le Coq, S. Aymerich, G. Gonzy-Tréboul, and P. Gay.** 1985. The DNA sequence of the gene for the secreted Bacillus subtilis enzyme levansucrase and its genetic control sites. Mol. Gen. Genet. **200**:220-228.

**35. Trieu-Cuot, P., and P. Courvalin.** 1983. Nucleotide sequence of the Streptococcus faecalis plasmid gene encoding the 3'5"-aminoglycoside phosphotransferase type III. Gene **23:331-341**.

**36.Vasantha, N., and L. D. Thompson.** 1986. Fusion of pro region of subtilisin to staphylococcal protein

A and its secretion by Bacillus subtilis. Gene **49**:23-28.

**37. Vieira, J., and J. Messing.** 1982. The pUC plasmid and M13mp7-derived system for insertion mutagenesis and sequencing with synthetic universal primers. Gene **19**:259-268.

**38. Wang.L. -F., S.-L. Wong, S.-G. Lee, N.K. Kalyan, P.P. Hung, S. Hilliker, and R. H. Doi.** 1988. Expression and secretion of human atrial natriuretic alpha-factor in Bacillus subtilis using the subtilisin signal peptide. Gene **69**:39-47.

**39. Zukowski, M. M., and L. Miller.** 1986. Hyperproduction of an intracellular heterologous protein in a sacU$^h$ mutant of Bacillus subtilis. Gene **46**:247-255.

**40. Te Riele, H., Michel, B., and Ehrlich, S.D.** 1986. Single-stranded plasmid DNA in Bacillus subtilis and Staphylococcus aureus. Proc. Natl. Acad. Sci. USA **83**:2541-2545.

**41. Niaudet, B., Jannière, L., and Ehrlich, S.D.** 1984. Recombination between repeated DNA sequences occurs more often in plasmids than in the chromosome of Bacillus subtilis. Mol. Gen. Genet. **197**:46-54.

**42. Young, M.** 1984. Gene amplification in Bacillus subtilis. J. Gen. Microbiol. **130**:1613-1621.

**43. Albertini, A.M., Galizzi, A.** 1985. Amplification of a chromosomal region on Bacillis subtilis. J. Bacteriol. **162**:1203-1211.

**44. Jannière, L., Niaudet, B., Pierre, E., and Ehrlich, S.D.** 1985. Stable gene amplification in the chromosome of Bacillus subtilis. Gene **40**:47-55.

**45. Kunst, F., Pascal, M., Lepesant-Kejzlarova, J., Lepesant, J.-A., Billault, A., et Dedonder, R.** 1974. Pleiotropic mutations affecting sporulation conditions and the syntheses of extracellular enzymes in Bacillus subtilis 168. Biochimie **56**:1481-1489.

**46. Weisblum, B., Graham, M. Y., Gryczan, T., and Dubnau, D.** 1979. Plasmid copy number control : isolation and characterization of high-copy-number mutants of plasmid pE194. J. Bacteriol. **137**:635-643.

**47. Noirot, Ph., Petit, M.A., and Ehrlich, S.D.** 1987. Plasmid replication stimulates DNA recombination in Bacillus subtilis. J. mol. Biol. **196**:39-48.

**48. Matsudaira, P.** 1987. Sequence from picomole quantities of proteins electroblotted onto polyvinylidene difluoride membranes. J. Biol. Chem. **262**:10035-10038.

**49. Shaw, W.V.** 1975. Chloramphenicol acetyltransferase from chloramphenicol-resistant bacteria. Methods Enzymol. **43**:737-755.

**50. Young, M., and Cullum, J.** 1987. A plausible mechanism for large-scale chromosomal DNA amplification in streptomycetes. FEBS lett. **212**:10-14.

**51. Pétré, D., Millet, J., Longin, R., Béguin, P. Girard, H., and Aubert, J.-P.** 1986. Purification and properties of the endoglucanase C of Clostridium thermocellum produced in Escherichia coli. Biochimie **68**:687-695.

**52. Béguin, P.** 1983. Detection of cellulase activity in polyacrylamide gels using congo red stained agar replicas. Anal. biochem. **131**:333-336.

**53. Dagert, M., Jones, I., Goze, A., Romac, S., Niaudet. B., and Ehrlich, S.D.** 1984. Replication functions of pC194 are necessary for efficient plasmid transduction of M13 phage. EMBO J. **3**:81-86.

**54. Niaudet, B., and Ehrlich, S.D.** 1979. In vitro genetic labeling of Bacillus subtilis cryptic plasmid pHV400. Plasmid **2**:48-58.

**55. Fouet, A., Klier, A., and Rapoport, G.** 1982. Cloning and expression in Escherichia coli of the sucrase gene from Bacillus subtilis. Mol. Gen. Genet. **186**:399-404.

**56.Stark, G.A., and G.M. Wahl.** 1984. Gene amplification. Ann. Rev. Biochem. 53:447-491

**57.Marrero, R. and Yasbin, R.E.** 1988. Cloning of the Bacillus subtilis recE gene and functional expression of recE in B. subtilis. J. Bacteriol. 170:335-344

**58.Sharf, S. J., Horn, G.T. and Erlich, H.A.** 1986. Science 233:1076-1078

**59.Williams et al.** 1981. J. Bacteriol. 146: 1162-1165

**60.Noirot, Ph., Petit, M.A., and S.D. Ehrlich.** 1987. Plasmid replication stimulates DNA recombination in Bacillus subtilis. J. Mol. Biol. 196: 39-48

**61.Tanaka and Sakaguchi.** 1978. Mol.Gen.Genet. 165: 269-276

**62.Yanish-Perron, C., Vieira, J. and Messing.** 1985. Gene 33: 103-119

## Revendications

**1.** Procédé de production d'une protéine déterminée caractérisé en ce qu'à partir d'une souche du genre Bacillus dans le chromosome de laquelle ont été intégrés dans un gène non essentiel de ladite souche :

a) une unité amplifiable comportant une séquence d'ADN déterminée M encadrée par deux séquences d'ADN identiques D orientées en sens direct, la séquence M comprenant le gène de structure de ladite protéine mis sous le contrôle d'éléments d'expression et le cas échéant sécrétion inductibles chez Bacillus de ladite protéine et facultativement un gène sélectionnable, et

b) un réplicon inductible ou conditionnel également intégré dans ledit gène non essentiel du chromosome de la souche en amont de l'unité amplifiable ;

1. on sélectionne éventuellement les clones amplifiés obtenus par culture sur un milieu de sélection correspondant au gène sélectionnable et l'on prélève les souches dont le génome correspond à la présence d'un nombre de copies dudit gène acrrû par rapport à la population bactérienne avant sélection ; et

2. dans un milieu de culture approprie, on induit ou conditionne une amplification gènique par l'activation du réplicon, et on induit l'expression et le cas échéant la sécrétion de la protéine par l'activation desdits éléments d'expression.

3. on récupère ladite protéine exprimée.

2. Procédé selon la revendication 1 caractérisé en ce que le gène codant pour la fonction de recombinaison homologue dans le chromosome de ladite souche est inactivé et/ou placé sous le contrôle d'un promoteur inductible, ledit gène codant pour la fonction de recombinason pouvant être un gène hétérologue lorsque le gène natif a été inactivé, de sorte que à l'étape 2, de la revendication 1 l'amplification ne se produit que si :

- on complémente le défaut de recombinaison homologue par un gène codant pour la fonction de recombinaison homologue dans le chromosome de ladite souche tel que le gène recE de Bacillus subtilis ou le gène recA de E. coli, et/ou
- dans le cas où ledit gène est placé sous le contrôle d'un promoteur inductible, on induit l'activation dudit promoteur inductible de sorte que la fonction de recombinaison homologue soit exprimée dans le chromosome de ladite souche.

3. Procédé selon la revendication 2 caractérisé en ce que le gène codant pour la fonction de recombinaison homologue est le gène natif recE de ladite souche de Bacillus et a été inactivé par transformation de ladite souche avec un gène recE muté, le gène recE natif étant éliminé par substitution de gène, notamment avec l'ADN chromosomique de la souche SBEK (qui dérive de la souche SB 202 de B. subtilis dans laquelle une insertion de 1.5 kb portant le gène aphA3 a été introduite dans la sequence codante du gène recE) et sélection du transformant résistant à la kanamycine.

4. Procédé de préparation d'une souche de Bacillus utile, notamment dans le procédé de production d'une protéine selon l'une des revendications 1 à 3 dans le chromosome de laquelle un gène déterminé codant ladite protéine a été intégré, éventuellement amplifié, caractérisé en ce que :

a) on effectue l'intégration dans le chromosome de ladite souche d'au moins une séquence d'ADN dite d'intégration comportant au moins :

. un réplicon inductible ou conditionnel en amont,

. une unité amplifiable de structure -D-M-D-, les séquences D orientées en sens direct assurant l'intégration du gène M dans un gène non essentiel de ladite souche, le gène M comprenant le gène de structure sde ladite protéine mis sous le contrôle d'éléments d'expression et le cas échéant sécrétion inductibles de ladite protéine chez Bacillus, l'unité amplifiable comportant en outre éventuellement un gène sélectionnable ;

b) On sélectionne éventuellement les souches de Bacillus obtenues par culture éventuellement sur un milieu de sélection correspondant au gène sélectionnable ; et

c) on prélève les souches stables dont le génome correspond à la présence d'un nombre de copies dudit gène accrû par rapport à la population bactérienne avant sélection.

5. Procédé selon la revendication 4 caractérisé en ce que le gène codant pour la fonction de recombinaison homologue dans le chromosome de ladite souche est inactivé et/ou placé sous le contrôle d'un promoteur inductible, ledit gène pouvant être le gène natif ou un gène hétérologue si le gène natif a été inactivé.

6. Procédé de préparation d'une souche de Bacillus utile notamment dans un procédé de production de protéines et en particulier un procédé selon l'une des revendications 2 ou 3, souche dans le chromosome de laquelle un gène déterminé codant pour ladite protéine a été intégré et amplifié, procédé caractérisé en ce que :

a) on effectue l'intégration dans le chromosome de ladite souche d'au moins une séquence d'ADN dite d'intégration comportant au moins :

. un réplicon inductible ou conditionnel en amont,

. une unité amplifiable de structure -D-M-D-, les séquences D orientées en sens direct assurant l'intégration du gène M dans un gène non essentiel de ladite souche, le gène M comprenant le gène de structure de ladite protéine mis sous le contrôle d'éléments d'expression et le cas échéant sécrétion inductibles de ladite protéine chez Bacillus, l'unité amplifiable comportant en outre éventuellement un gène sélectionnable ;

b) On sélectionne éventuellement les souches de Bacillus obtenues par culture éventuellement sur un milieu de sélection correspondant au gène sélectionnable ;

c) dans un milieu de culture appropriée, on induit une amplification génique par l'activation du réplicon:

d) on bloque l'expression de la fonction de recombinaison homologue dans le chromosome de ladite souche ; et

e) on prélève les souches stables dont le génome correspond a la présence d'un nombre de copies dudit gène accrû par rapport à la population bactérienne avant sélection.

7. Procédé selon la revendication 6 caractérisé en ce que le contrôle de l'expression de la fonction de recombinaison homologue a l'étape c) se fait :

- en plaçant le gène codant pour la fonction de recombinaison homologue dans le chromosome de ladite souche sous le contrôle d'un promoteur inductible, ledit gène pouvant être le gène natif ou le gène hétérologue si le gène natif a été inactivé, auquel cas à l'étape c) on induit également l'activation dudit promoteur du gène codant pour la fonction de recombinaison et à l'étape d) on interrompt l'induction de l'activation dudit promoteur,

ou

- en transformant ladite souche avec un gène recE muté, le gène recE natif étant éliminé par substitution de gène, notamment avec l'ADN chromosomique de la souche SBEK (qui dérive de la souche SB 202 de B. subtilis dans laquelle une insertion de 1.5 kb portant le gène aphA3 a été introduite dans la séquence codante du gène recE) et sélection des transformants résistants à la kanamycine.

8. Procédé de préparation d'une protéine déterminée caractérisé en ce que on cultive une souche selon l'une des revendications 6 ou 7, dans laquelle le gène déterminé code pour ladite protéine, dans un milieu de culture et en ce que l'on sépare ensuite la protéine exprimée et le cas échéant secrétée.

9. Procédé selon l'une des revendications précédentes, caractérisé en ce que le réplicon provient d'un plasmide bactérien.

10. Procédé selon l'une des revendications précédentes, caractérisé en ce que l'activation du réplicon est thermosensible.

11. Procédé selon la revendication 10, caractérisé en ce que le réplicon provient du plasmide pE194.

12. Procédé selon l'une des revendications précédentes, caractérisé en ce que la souche est une souche de Bacillus subtilis, Bacillus alcalophilus, Bacillus brevis, Bacillus circulans, Bacillus lentus, Bacillus licheniformis, Bacillus macerans, Bacillus megaterium, Bacillus polymyxa, Bacillus pumilus, Bacillus stearothermophilus, Bacillus coagulans.

13. Procédé selon l'une des revendications précédentes, caractérisé en ce que le système de réplication et l'unite amplifiable DMD sont intégrés entre les séquences thy B et X après délétion du gène ilvA de Bacillus subtilis.

14. Procédé selon l'une des revendications précédentes, caractérisé en ce que les séquences D proviennent d'un plasmide bactérien tel que le plasmide pBR322.

15. Procédé selon l'une des revendications précédentes, caractérisé en ce que le gène sélectionnable est un gène de résistance à un composé chimique tel que le chloramphénicol ou la kanamycine.

16. Procédé selon l'une des revendications précédentes, caractérisé en ce que les éléments d'expression et de sécrétion de Bacillus subtilis consistent dans le promoteur sacB accompagné de la région sacR inductible par le saccharose.

17. Procédé selon la revendication 16, caractérisé en ce que les éléments d'expression et de sécrétion chez

Bacillus subtilis consistent en la partie du locus sacRB de Bacillus subtilis qui contient
    1) le locus sacR contenant le promoteur et la région de régulation du gène sacB, et
    2) la région de sacB codant la séquence signal de la lévane-saccharase.

**18.** Procédé selon l'une des revendications précédentes, caractérisé en ce que la souche est une souche mutante sacU^h.

**19.** Procédé selon l'une des revendications précédentes, caractérisé en ce qu'on récupère la protéine exprimée lorsque la culture est en phase stationnaire.

**20.** Procédé selon l'une des revendications précédentes, caractérisé en ce que ladite protéine est l'endoglucanase A (EGA) dont le gène de structure est le gène celA isolé chez Clostridium thermocellum qui est mis sous le contrôle d'éléments d'expression et de sécrétion chez Bacillus subtilis.

**21.** Souches obtenues par le procédé de préparation de souches selon l'une des revendications précédentes.

## Claims

**1.** Process for the preparation of a determined protein, characterized in that starting with a strain of the Bacillus genus in whose chromosome there have been integrated into a non-essential gene of the said strain:
    a) an amplifiable unit containing a determined DNA sequence M flanked by two identical DNA sequences D orientated in the direct orientation, the M sequence comprising the structural gene of the said protein placed under the control of elements for expression and, where appropriate, secretion, which are inducible in Bacillus, of the said protein and, optionally, a selectable gene, and
    b) an inducible or conditional replicon, also integrated into the said non-essential gene of the chromosome of the strain upstream of the amplifiable unit;
        1. the amplifiable clones obtained are optionally selected by culturing on a selection medium corresponding to the selectable gene and the strains whose genome corresponds to the presence of an increased number of copies of the said gene relative to the bacterial population before selection, are recovered; and
        2. in an appropriate culture medium, a gene amplification is induced or conditioned by activation of the replicon and the expression and, where appropriate, the secretion of the protein is induced by activation of the said expression elements.
        3. the said expressed protein is recovered.

**2.** Process according to Claim 1, characterized in that the gene encoding the homologous recombination function in the chromosome of the said strain is inactivated and/or placed under the control of an inducible promoter, it being possible for the said gene encoding the recombination function to be a heterologous gene when the native gene has been inactivated, such that in stage 2. of Claim 1, the amplification occurs only if:
    - the homologous recombination defect is complemented by a gene encoding the homologous recombination function in the chromosome of the said strain such as the recE gene of Bacillus subtilis or the recA gene of E. coli, and/or
    - in the case where the said gene is placed under the control of an inducible promoter, the activation of the said inducible promoter is induced such that the homologous recombination function is expressed in the chromosome of the said strain.

**3.** Process according to Claim 2, characterized in that the gene encoding the homologous recombination function is the native recE gene of the said Bacillus strain and has been inactivated by transformation of the said strain with a mutated recE gene, the native recE gene being removed by gene substitution, especially with chromosomal DNA of the SBEK strain (which is derived from the strain SB202 of B. subtilis in which a 1.5 kb insert carrying the aPhA3 gene has been introduced into the sequence encoding the recE gene) and selection of the kanamycin-resistant transformant.

**4.** Process for the preparation of a Bacillus strain useful especially in the process for the production of a protein according to one of Claims 1 to 3, in whose chromosome a determined gene encoding the said protein has been integrated, optionally amplified, characterized in that:
    a) there is integrated into the chromosome of the said strain at least one so-called integrative DNA sequence containing at least:

  . one inducible or conditional replicon upstream,

  . one amplifiable unit of structure -D-M-D-, the D sequences orientated in the direct orientation ensuring the integration of the M gene into a non-essential gene of the said strain, the M gene comprising the structural gene of the said protein placed under the control of inducible elements for expression and, where appropriate, secretion of the said protein in Bacillus, the amplifiable unit containing, in addition, optionally, a selectable gene;

b) the Bacillus strains obtained are optionally selected by culturing, optionally on a selection medium corresponding to the selectable gene; and

c) the stable strains whose genome corresponds to the presence of an increased number of copies of the said gene relative to the bacterial population before selection, are recovered.

5. Process according to Claim 4, characterized in that the gene encoding the homologous recombination function in the chromosome of the said strain is inactivated and/or placed under the control of an inducible promoter, it being possible for the said gene to be the native gene or a heterologous gene if the native gene has been inactivated.

6. Process for the preparation of a Bacillus strain useful especially in a process for the production of proteins and in particular a process according to one of Claims 2 or 3, in the chromosome of which strain a determined gene encoding the said protein has been integrated and amplified, which process is characterized in that:

a) there is integrated into the chromosome of the said strain at least one so-called integrative DNA sequence containing at least:

  . one inducible or conditional replicon upstream,

  . one amplifiable unit of structure -D-M-D-, the D sequences orientated in the direct orientation ensuring the integration of the M gene into a non-essential gene of the said strain, the M gene comprising the structural gene of the said protein placed under the control of inducible elements for expression and, where appropriate, secretion of the said protein in Bacillus, the amplifiable unit containing, in addition, optionally, a selectable gene;

b) the Bacillus strains obtained are optionally selected by culturing, optionally on a selection medium corresponding to the selectable gene;

c) in an appropriate culture medium, a gene amplification is induced by activation of the replicon;

d) the expression of the homologous recombination function in the chromosome of the said strain is blocked; and

e) the stable strains whose genome corresponds to the presence of an increased number of copies of the said gene relative to the bacterial population before selection, are recovered.

7. Process according to Claim 6, characterized in that the control of the expression of the homologous recombination function in stage c) is performed:

- by placing the gene encoding the homologous recombination function in the chromosome of the said strain under the control of an inducible promoter, it being possible for the said gene to be the native gene or the heterologous gene if the native gene has been inactivated, in which case in stage c), the activation of the said promoter for the gene encoding the recombination function is also induced and in stage d) the induction of the activation of the said promoter is interrupted, or

- by transforming the said strain with a mutated recE gene, the native recE gene being removed by gene substitution, especially with chromosomal DNA of the SBEK strain (which is derived from the strain SB202 of B. subtilis in which a 1.5 kb insert carrying the aPhA3 gene has been introduced into the sequence encoding the recE gene) and selection of the kanamycin-resistant transformants.

8. Process for the preparation of a determined protein, characterized in that a strain according to one of Claims 6 or 7, in which the determined gene encodes the said protein, is cultured in a culture medium, and in that the protein expressed and, where appropriate, secreted is then separated.

9. Process according to one of the preceding claims, characterized in that the replicon is obtained from a bacterial plasmid.

10. Process according to one of the preceding claims, characterized in that the activation of the replicon is heat-sensitive.

11. Process according to Claim 10, characterized in that the replicon is obtained from the plasmid pE194.

12. Process according to one of the preceding claims, characterized in that the strain is a strain of <u>Bacillus subtilis, Bacillus alcalophilue, Bacillus brevis, Bacillus circulans, Bacillus lentus, Bacillus licheniformis, Bacillus macerans, Bacillus megaterium, Bacillus polymyxa, Bacillus pumilus, Bacillus stearothermophilus, Bacillus coagulans</u>.

13. Process according to one of the preceding claims, characterized in that the replication system and the amplifiable unit DMD are integrated between the sequences <u>thy</u> B and X after deletion of the <u>ilvA</u> gene of <u>Bacillus subtilis</u>.

14. Process according to one of the preceding claims, characterized in that the D sequences are obtained from a bacterial plasmid such as the plasmid pBR322.

15. Process according to one of the preceding claims, characterized in that the selectable gene is a gene for resistance to a chemical compound such as chloramphenicol or kanamycin.

16. Process according to one of the preceding claims, characterized in that the expression and secretion elements of <u>Bacillus subtilis</u> consist of the promoter <u>sacB</u> accompanied by the <u>sacR</u> region inducible by sucrose.

17. Process according to Claim 16, characterized in that the expression and secretion elements in <u>Bacillus subtilis</u> consist of the part of the <u>sacRB</u> locus of <u>Bacillus subtilis</u> which contains
1) the <u>sacR</u> locus containing the promoter and the regulatory region of the <u>sacB</u> gene, and
2) the <u>sacB</u> region encoding the signal sequence of levan saccharase.

18. Process according to one of the preceding claims, characterized in that the strain is a mutant strain <u>sacU</u>[h].

19. Process according to one of the preceding claims, characterized in that the expressed protein is recovered when the culture is in the stationary phase.

20. Process according to one of the preceding claims, characterized in that the said protein is endoglucanase A (EGA) whose structural gene is the <u>celA</u> gene isolated from <u>Clostridium thermocellum</u> which is placed under the control of expression and secretion elements in <u>Bacillus subtilis</u>.

21. Strains obtained by the process for the preparation of strains according to one of the preceding claims.

## Patentansprüche

1. Verfahren zur Herstellung eines bestimmten Proteins, dadurch gekennzeichnet, daß man, ausgehend von einem Stamm der Gattung <u>Bacillus</u>, in dessen Chromosom in ein nicht essentielles Gen des Stamms integriert wurden:
a) eine amplifizierbare Einheit, umfassend eine bestimmte DNA-Sequenz M, die umgeben ist von zwei im direkten Sinn angeordneten DNA-Sequenzen D, wobei die Sequenz M ein Strukturgen des Proteins umfaßt, das kontrolliert wird von Expressionselementen und gegebenenfalls bei <u>Bacillus</u> induzierbaren Sekretionselementen des Proteins und wahlweise ein Selektionsmarker und
b) oberhalb der amplifizierbaren Einheit ein induzierbares oder konditionierbares Replikon (réplicon conditionnel), das gleichzeitig integriert ist in das nicht essentielle Gen des Chromosoms des Stamms;
1. gegebenenfalls die amplifizierten Klone selektiert, die erhalten wurden durch Kultur auf einem für den Selektionsmarker geeigneten Kulturmedium, und man die Stämme entnimmt, deren Genom der Anwesenheit einer verglichen mit der Bakterienpopulation vor der Selektion vermehrten Anzahl von Kopien des besagten Gens entspricht; und
2. in einem geeigneten Kulturmedium durch Aktivierung des Replikons eine genetische Amplifikation induziert oder konditioniert, und man gegebenenfalls die Sekretion des Proteins durch Aktivierung der Expressionselemente induziert,

3. das exprimierte Protein isoliert.

2. Verfahren nach Anspruch 1, dadurch gekennzeichnet, daß das für die homologe Rekombinationsfunktion codierende Gen im Chromosom des Stamms inaktiviert ist oder von einem induzierbaren Promoter kontrolliert wird, wobei das für die Rekombinationsfunktion codierende Gen ein heterologes Gen sein kann, wenn das native Gen inaktiviert wurde, so daß in der 2. Stufe von Anspruch 1 die Amplifikation nicht stattfindet außer:

- man behebt den Mangel der homologen Rekombination durch ein Gen, das für die homologe Rekombinatinsfunktion im Chromosom des Stammes codiert, wie das Gen recE von Bacillus subtilis oder das Gen recA von E. coli, und/oder
- man induziert die Aktivierung des Promoters, so daß die homologe Rekombinationsfunktion im Chromosom des Stamms exprimiert wird, falls das Gen von einem induzierbaren Promoter kontrolliert wird.

3. Verfahren nach Anspruch 2, dadurch gekennzeichnet, daß es sich bei dem für die homologe Rekombinationsfunktion codierenden Gen um das native Gen recE des Stamms Bacillus handelt, das durch Transformation des Stamms mit einem mutierten Gen recE inaktiviert wurde, wobei das native Gen recE durch Substitution des Gens entfernt wurde, nämlich mit chromosomaler DNA des Stamms SBEK, der abstammt vom Stamm SB202 des B. subtilis, in den in die für das Gen recE codierende Sequenz eine Insertion von 1.5 kB eingeführt wurde, die das Gen aphA3 trägt, und die gegen Kanamycin resistenten Transformanden selektiert wurden.

4. Verfahren zur Herstellung eine nützlichen Stammes von Bacillus, nämlich im Herstellungsverfahren für ein Protein nach einem der Ansprüche 1 bis 3, in dessen Chromosom eine bestimmtes für das Protein codierendes Gen integriert wurde, gegebenfalls amplifiziert, dadurch gekennzeichnet, daß:

a) man die Integration von mindestnes einer DNA-Sequenz in das Chromosom des Stamms durchführt, wobei die Integration mindestens umfaßt:
. ein induzierbares oder konditionierbares Replikon (réplicon conditionnel) oberhalb,
. eine amplifizierbare Einheit der Struktur -D-M-D-, wobei die Sequenzen D im direkten Sinn orientiert sind und die Integration des Gens M in ein nicht essentielles  Gen des Stamms gewährleisten, und wobei das Gen M das Strukturgen des Proteins umfaßt, kontrolliert von Expressionselementen und gegebenenfalls bei Bacillus induzierbaren Sekretionselementen des Proteins, wobei die amplifizierbare Einheit gegebenenfalls zusätzlich einen Selektionsmarker umfaßt;
b) man gegebenfalls die durch Kultur, gegebenenfalls auf einem für den Selektionsmarker geeigneten Kulturmedium, erhaltenen Stämme von Bacillus selektiert; und
c) man die stabilen Stämme entnimmt, deren Genom einer im Vergleich zur Bakterienpopulation vor der Selektion vermehrten Anzahl von Kopien des Gens entspricht.

5. Verfahren nach Anspruch 4, dadurch charakterisiet, daß das für die homologe Rekombinationsfunktion codierende Gen im Chromosom des Stamms inaktiviert ist und/oder von einem induzierbaren Promoter kontrolliert wird, wobei das Gen ein natives Gen oder ein heterologes Gen sein kann, falls das native Gen inaktiviert wurde.

6. Verfahren zur Herstellung eines nützlichen Stammes von Bacillus, nämlich in einem Verfahren zur Herstellung von Proteinen insbesonder im Verfahren nach einem der Ansprüche 2 oder 3, eines Stammes, in dessen Chromosom ein bestimmtes, für das Protein codierende Gen integriert und amplifiziert wurde, wobei das Verfahren dadurch gekennzeichnet ist, daß:

a) man die Integration von mindestens einer DNA-Sequenz in das Chromosom des Stammes durchführt, wobei die Integration mindestens umfaßt:
. ein induzierbares oder konditionales Replikon oberhalb,
. eine amplifizierbare Eineit der Struktur -D-M-D, wobei die Sequenzen D im direkten Sinn angeordnet sind und die Integration des Gens M in ein nicht essentielles Gen des Stamms gewährleisten, und wobei das Gen M das Strukturgen des Proteins umfaßt, kontrolliert von Expressionselementen und gegebenenfalls bei Bacillus induzierbaren Sekretionselementen, und wobei die amplifizierbare Einheit gegebenenfalls einen Selektionsmarker umfaßt;
b) man gegebenenfalls die durch Kultur, gegebenenfalls auf einem für den Selektionsmarker geeigneten Kulturmedium, erhaltenen Stämme selektiert;
c) man in einem geeigneten Kulturmedium eine genetische Amplifikation induziert durch Aktivierung des Replikons;

d) man die Expression der homologen Rekombinationsfunktion im Chromosom des Stamms blockiert; und

e) man die stabilen Stämme entnimmt, deren Genom der Anwesenheit einer im Vergleich zu der Bakterienpopulation vor der Selektion erhöhten Anzahl von Kopien des Gens entspricht.

7.  Verfahren nach Anspruch 6, dadurch gekennzeichnet, daß die Kontrolle der Expression der homologen Rekombinationsfunktion im Schritt c) wie folgt erfolgt:

- indem das für die homologe Rekombinationsfunktion codierende Gen im Chromosom des Stammes so plaziert wird, daß es der Kontrolle eines induzierbaren Promoters unterliegt, wobei das Gen das native Gen sein kann oder das heterologe Gen, falls das native Gen inaktiviert wurde, wobei im letzteren Fall im Schritt c) gleichermaßen eine Aktivierung des Promoters des für die homologe Rekombinationsfunktion codierenden Gens induziert wird, und im Schritt d) die Induktion der Aktivierung des Promoters unterbrochen wird, oder

- man den Stamm mit einem mutierten Gen recE transformiert, wobei das native Gen recE durch Substitution des Gens eliminiert wurde, nämlich mit chromosomaler DNA aus dem Stamm SBEK, abstammend vom Stamm SB202 von B. subtilis, in dessen für das Gen recE codierende Sequenz eine Insertion von 1,5 kB eingeführt wurde, die das Gen aphA3 trägt, und die gegen Kanamycin resistenten Transformanden selektiert wurden.

8.  Verfahren zur Herstellung eines bestimmten Proteins, dadurch gekennzeichnet, daß man einen Stamm nach einem der Annsprüche 6 oder 7, in dem das bestimmte Gen für das Protein codiert, in einem Kulturmedium kultiviert und anschließend das exprimierte und gegebenenfalls sezernierte Protein abtrennt.

9.  Verfahren nach einem der vorstehenden Ansprüche, dadurch gekennzeichnet, daß das Replikon aus einem bakteriellen Plasmid stammt.

10. Verfahren nach einem der vorstehenden Ansprüche, dadurch gekennzeichnet, daß die Aktivierung des Replikons thermosensibel ist.

11. Verfahren nach Anspruch 10, dadurch gekennzeichnet, daß das Replikon vom Plasmid pE194 stammt.

12. Verfahren nach einem der vorstehenden Ansprüche, dadurch gekennzeichnet, daß es sich bei dem Stamm um einen Stamm von Bacillus subtilis, Bacillus alcalophilus, Bacillus brevis, Bacillus circulans, Bacillus lentus, Bacillus licheniformis, Bacillus macerans, Bacillus megaterium, Bacillus polymyxa, Bacillus pumilus, Bacillus stearothermophilus, Bacillus coagulans handelt.

13. Verfahren nach einem der vorstehenden Ansprüche, dadurch gekennzeichnet, daß das Replikationssystem und die amplifizierbare Einheit DMD zwischen die Sequenzen thy B und X integriert werden nach Deletion des Gens ilvA von Bacillus subtilis.

14. Verfahren nach einem der vorstehenden Ansprüche, daurch gekennzeichnet, daß die Sequenz D aus einem bakteriellen Plasmid wie dem Plasmid pBR322 stammt.

15. Verfahren nach einem der vorstehenden Ansprüche, dadurch gekennzeichnet, daß es sich bei dem Selektionsmarker um ein Resistenzgen gegen eine chemische Verbindung wie Chloramphenicol oder Kanamycin handelt.

16. Verfahren nach einem der vorstehenden Ansprüche, dadurch gekennzeichnet, daß die Expressions- und Sekretionselemente von Bacillus subtilis aus einem Promoter sacB, begleitet von einer durch Saccharose induzierbaren Region sacR bestehen.

17. Verfahren nach Anspruch 16, dadurch gekennzeichnet, daß die Expressions- und Sekretionselemente von Bacillus subtilis aus einem Teil des Locus sacRB von Bacillus subtilis stammen, der enthält:

1) den Locus sacR, enthaltend den Promoter der regulierenden Region des Gens sacB, und

2) die Region von sacB, die für die Signalsequenz der Lävansaccharase codiert.

18. Verfahren nach einem der vorstehenden Ansprüche, dadurch gekennzeichnet, daß es sich bei dem Stamm um einen Mutanten-Stamm sacU$^h$ handelt.

19. Verfahren nach einem der vorstehenden Ansprüche, dadurch gekennzeichnet, daß man das exprimierte Protein gewinnt, während die Kultur in stationärem Zustand ist.

20. Verfahren nach einem der vorstehenden Ansprüche, dadurch gekennzeichnet, daß es sich bei dem Protein um Endoglucanase A (EGA) handelt, deren Strukturgen das bei Clostridium thermocellum isolierte Gen ceiA ist, das kontrolliert wird von Expressions- und Sekretionselementen bei Bacillus subtilis.

21. Stämme, erhalten nach dem Verfahren zur Herstellung von Stämmen nach einem der vorstehenden Ansprüche.

Tableau 1 : plasmides et souches

| plasmide et souche | Genotype | phenotype [*] | Reference |
|---|---|---|---|
| *B. subtilis* | | | |
| 168 | *trpC2* | Lvs[+] | 21 |
| QB 25 | *trpC2 sacS$^C$49* | Lvs[C] | ce travail |
| QB136 | *leuA8 trpC2 sacU$^h$32* | Lvs[h] | * |
| QB907 | *trpC2 sacA321 sacS$^h$3* | Lvs[h] | * |
| QB671 | *hisA1 leuA8 thr-5 sacS$^h$3* | Lvs[h] | * |
| *E. coli* | | | |
| TG1 | *K12, Δ( lac-pro), supE, thi hsdD5/F' traD36, proA B lacI , lacZΔM15* | *rk− mk− repair+* | 13 |
| HB2154 | *K12, ara, Δ(lac-pro), thi/F' proA B , lacI , lacZΔM15, mutL::Tn10* | *rk+ mk+ repair−* | 8 |
| **Plasmids** | | | |
| pUC8 | | Ap[r] | 38 |
| pC194 | | Cm[r] | 16 |
| pCT105 | *celA* | Ap[r] Tc[r] | 10 |
| pAE101 | *sacR ΔsacB* | Km[r] Ap[r] | 11 |
| pBS413 | *sacR sacB* | Ap[r] | 1 |
| pBS430 | *sacR ΔsacB* | Ap[r] Cm[r] | 17 |

*Lvs, lévane-saccharase extracellulaire. Lvs[+], production de type
sauvage ; Lvs[C], constitutive production , Lvs[h] hyperproduction

## FIG_1

Tableau 2 : Activité CMC-ase dans des surnageants de culture de
différentes souches transformées de <u>Bacillus subtilis</u>

| Souches | phenotype | Plasmid | Induction par saccharose | Activité CMCase (Unités/ml) |
|---|---|---|---|---|
| 168 | type sauvage | pC194 | + | < 0.01 |
| " | " | pGJ2 | - | < 0.01 |
| " | " | " | + | 0.10 |
| QB25 | SacS$^c$ | pC194 | + | < 0.01 |
| " | " | pGJ2 | - | 0.11 |
| " | " | " | + | 0.38 |
| QB907 | SacS$^h$ | pC194 | + | 0.01 |
| " | " | pGJ2 | - | 0.01 |
| " | " | " | + | 0.56 |
| QB136 | SacU$^h$ | pC194 | + | 0.02 |
| " | " | pGJ2 | - | 0.02 |
| " | " | " | + | 0.80 |

* L'activité est mesurée dans des surnageants de culture récupérés en fin de phase
logarithmique

# FIG_2

FIG.3

EP 0 410 895 B1

FIG.4

DGF3 (30 mer)          3' TGAGTTCGCAAACGG CGTCCACACGGAAAA 5'
pGJ3 (strand+) 5'-GCGCAACTCAAGCGTTTGCC GCAGGTGTGCCTTTTAACAC-3'
                ----- séquence signal ──→  ←── séquence mature
                           Lvs          ┌G  G            EGA
                               Nae I    │G  C
                                        └C  G
                                         A  A
                                         A  C
                                         A  T
                                         C  G
                                         A  T
                                         CTG

                              ↑
                19 nucleotides de la
                séquence signal de l'EGA

FIG_5

FIG_6

EP 0 410 895 B1

FIG_7

Tableau 3 : Souches et plasmides

| Souche et plasmide | Genotype | phenotype | Reference |
|---|---|---|---|
| **E. coli** | | | |
| TG1 | K12, $\Delta$(lac-pro), supE, thi hsdD5/F' traD36,ProA⁺ B⁺ lacIᵠ, lacZΔM15 | | 1 8 |
| **B. subtilis** | | | |
| QB136 | leuA8 trpC2 sacU$^h$32 | SacU$^h$ | ce travail |
| APO | tyrA1 aroB2 hisH2 trpC2 ins(pAD2) del(ilvA) | Em$^r$ | S.D. Ehrlich |
| AP551 | tyrA1 aroB2 hisH2 trpC2 ins(pAD2) del(ilvA) SacU$^h$32 ins(pHV551) dup(pBR322Δ81) | SacU$^h$CMC$^+$ Em$^r$ Cm$^r$ | ce travail |
| **Plasmids** | | | |
| pGJ5 | sacR ΔsacB celA | Ap$^r$ CMC$^+$ | 2 |
| pHV33Δ81 | | Ap$^r$ Cm$^r$ | 1 9 |
| pHV551 | sacR ΔsacB celA | Ap$^r$ Cm$^r$ CMC$^+$ | ce travail |

## FIG_8

FIG_9

FIG.10

FIG.11

EP 0 410 895 B1

45%      25%

--- Gly - Ala - Thr - Gln - Ala - Phe - Ala - Ala - Gly - Val - Pro - Phe - Asn - - -

-----Signal sequence Lvs ———→ ←——— Mature sequence EGA-----

30%

FIG_12

FIG_13

## FIGURE 14

HEURES

△   DENSITE OPTIQUE (660 nm)

□   ACTIVITE EGA ( U/ML )

●   ACTIVITE SPECIFIQUE CAT ( U/MG ) × $10^{-2}$

○   NOMBRE DE COPIES DE L'UNITE D'AMPLIFICATION × $10^{-1}$

FIGURE 15 : A

FIGURE 15 : B